# EUROPÄISCHE PATENTSCHRIFT

(11) **EP 0 679 619 B1**
(45) Veröffentlichungstag und Bekanntmachung des Hinweises auf die Patenterteilung: **17.10.2001**
(21) Anmeldenummer: 95105545.8
(22) Anmeldetag: 12.04.1995
(51) Int. Cl.: C07B 37/04, C07C 255/50, C07D 401/04, C07D 401/14

(54) **Verfahren zur Kreuzkupplung von aromatischen Borverbindungen mit aromatischen Halogenverbindungen oder Perfluoralkylsulfonaten**
Process for cross-coupling of aromatic boron compounds with aromatic halogen compounds or perfluoroalkyl sulphonates
Procédé de couplage croisé de composés aromatiques du bore avec des composés aromatiques halogénés ou de sulphates de peralkyle

(30) Priorität: 26.04.1994 DE 4414499
(43) Veröffentlichungstag der Anmeldung: 02.11.1995
(73) Patentinhaber: Aventis Research & Technologies GmbH & Co. KG, 65926 Frankfurt am Main (DE)
(72) Erfinder: Haber, Steffen, Dr., D-76726 Germersheim (DE); Manero, Javier, Dr., D-65931 Frankfurt (DE)
(74) Vertreter: Isenbruck, Günter, Dr.

(56) Entgegenhaltungen:
- WO-A-94/00423
- WO-A-94/10105
- DE-A- 4 307 243
- DE-C- 3 930 663

## Beschreibung

Die Erfindung betrifft ein Verfahren zur Herstellung mehrkerniger aromatischer Verbindungen durch Kreuzkupplung von aromatischen Borverbindungen mit aromatischen Halogenverbindungen oder Perfluoralkylsulfonaten unter Katalyse durch metallisches Palladium.

Die palladiumkatalysierte Kreuzkupplungsreaktion von terminalen Alkinen und metallorganischen Alkyl-, Alkenyl-, Allyl- oder Arylverbindungen mit Alkyl-, Alkenyl-, Allyl- oder Arylhalogeniden bzw. - Perfluoralkylsulfonaten wird seit einigen Jahren in steigendem Umfang in vielen Bereichen der organischen Synthese genutzt (siehe z.B. B.M. Trost, T.R. Verhoeven in: Comprehensive Organometallic Chemistry Volume 8, S. 779 ff., Pergamon Press, Oxford 1982).

Auch aromatische Borverbindungen, wie Boronsäuren und deren Derivate oder Borane, wurden zur Kupplung mit aromatischen Halogenverbindungen bzw. - Perfluoralkylsulfonaten eingesetzt (siehe z.B. N. Miyaura, T. Yanagi, A. Suzuki in Synthetic Communications 11 (1981) 513; EP-A 0 470 795 und EP-A 0 354 434).

Bei den dort beschriebenen Verfahren handelt es sich um homogen katalysierte Prozesse unter Verwendung von Pd(0)-Komplexen, insbesondere Tetrakis-(triphenylphosphan)-palladium(0), die in organischen Lösungsmitteln löslich sind.

Der Nachteil dieser Verfahren liegt jedoch eindeutig bei den hohen Katalysatorkosten, die eine wirtschaftliche Überführung der Prozesse in einen größeren Produktionsmaßstab (kg, t) schwierig machen. Die homogene Reaktionsführung erschwert desweiteren eine effiziente Regenerierung des Palladium-Katalysators und kann leicht zu einer Kontamination der bei der Reaktion anfallenden Produkte wie auch des Abfalls mit Palladium führen.

Es ist auch bekannt, wasserlösliche Palladiumkomplexe für die obengenannten Kupplungsreaktionen einzusetzen und in rein wäßrigen- oder Zweiphasensystemen aus organischer - und Wasserphase zu arbeiten (siehe z.B. US 5,043,510; A.L. Casalnuovo und J.C. Calabrese, J. Am. Chem. Soc. 112 (1990) 4324 und J.P. Genet et al., Synlett 1992, 715). Dabei werden wasserlösliche Phosphanliganden, wie Triphenylphosphano-3,3',3",-trisulfonattrinatriumsalz (tppts), verwendet, um den wasserlöslichen Palladiumkomplex zu erhalten.

Die in wäßriger Phase rein homogen geführten Prozesse leiden jedoch ebenso unter den oben beschriebenen Nachteilen und für die Zweiphasensysteme wird ein Verschleppen des Palladiums in die organische Phase beobachtet.

Auch in der WO 94 00423 ist ein homogenkatalytisches Verfahren beschrieben, mit dem einkernige aromatische Verbindungen hergestellt werden. Dies geschieht durch Kreuzkupplung von Boronsäuren mit Halogenverbindungen unter Katalyse durch Palladiumverbindungen, wobei dieses Komplexe von Palladium (II) oder Palladium (0) sein können. Das Verfahren ist auf die Herstellung einkerniger aromatischer Verbindungen beschränkt.

Es sind deshalb Verfahren entwickelt worden, die diese Probleme durch heterogenen Einsatz des Katalysators umgehen.
In der deutschen Patentschrift 39 30 663 ist ein Verfahren zur Herstellung flüssigkristalliner Verbindungen beschrieben, bei dem Halogenide und metallorganische Verbindungen, darunter auch Boronsäuren, in inerten Lösungsmitteln unter Verwendung von metallischem, ggf. auf ein Trägermaterial aufgezogenem Palladium als Katalysator, ggf. in Gegenwart eines Metallalkoholats umgesetzt werden. Durch diese Vorgehensweise können zwar die Katalysatorkosten wesentlich gesenkt und das Palladium kann nach Beendigung der Umsetzungen leicht regeneriert werden, jedoch liefert dieses Verfahren das angestrebte Kupplungsprodukt nicht immer in befriedigenden Ausbeuten und genügender Reinheit.

Die DE-43 04 243 offenbart ein Verfahren zur Herstellung mehrkerniger aromatischer Verbindungen durch Kreuzkupplung von aromatischen Boronsäuren oder deren Derivaten mit aromatischen Halogenverbindungen oder Fluoralkylsulfonaten unter Katalyse durch metallisches Palladium auf einem Trägermaterial. In Beispiel 1 findet sich dabei die Offenbarung eines Verfahrens zur Herstellung von 4'-Fluor-4-diphenyl-carboxaldehyd, indem p-Brombenzaldehyd mit p-Fluorphenylboronsäure umgesetzt wird. Als Katalysator findet metallisches Palladium auf Aktivkohle Verwendung, das Lösungsmittel ist ein Gemisch aus 40 % Benzol, 20 % Ethanol und 40 % NaCO₃-Lösung.

Aufgabe der vorliegenden Erfindung war es somit, ein Verfahren zur Kupplung von aromatischen Borverbindungen mit aromatischen Halogenverbindungen oder Perfluoralkylsulfonaten zu finden, das die beschriebenen Nachteile zumindest teilweise behebt.

Es wurde nun überraschend gefunden, daß bei Umsetzung von aromatischen Borverbindungen, wie Boronsäuren, mit aromatischen Halogenverbindungen oder -Perfluoralkylsulfonaten in einem wäßrigen Reaktionsmedium in Gegenwart einer Base, katalytischer Mengen an metallischem Palladium und mindestens eines wasserlöslichen Komplexliganden mehrkernige aromatische Verbindungen in hervorragenden Ausbeuten und sehr hohen Reinheiten erhalten werden.

Gegenstand der Erfindung ist somit ein Verfahren zur Herstellung mehrkerniger aromatischer Verbindungen durch Kreuzkupplung von aromatischen Borverbindungen mit aromatischen Halogenverbindungen oder -Perfluoralkylsulfonaten in Gegenwart von metallischem Palladium als Katalysator und soviel Wasser, daß die Reaktionsmischung eine wäßrige Phase ausbildet, dadurch gekennzeichnet, daß man der Reaktion mindestens einen wasserlöslichen Komplexliganden zusetzt.

Die erfindungsgemäße Reaktion verläuft chemoselektiv, so daß selbst elektrophile Gruppen, wie Ester oder Nitrile, den Verlauf der Reaktion nicht beeinträchtigen.

Durch den erfindungsgemäßen Einsatz eines wasserlöslichen Komplexliganden in wäßriger Phase lassen sich mehrkernige aromatische Verbindungen in sehr guten Ausbeuten bei gleichzeitig sehr hoher Reinheit, insbesondere ohne Verunreinigung durch die Komplexliganden, herstellen.

Darüber hinaus läßt sich das nach Beendigung der Reaktion als Feststoff vorliegende Palladiummetall leicht abtrennen, regenerieren und wieder dem Katalyseprozeß zuführen, wodurch eine zusätzliche Senkung der Verfahrenskosten und eine Entlastung der Abfallprodukte von Palladium erreicht wird.

Zur Durchführung des erfindungsgemäßen Verfahrens werden die aromatische Borverbindung, die aromatische Halogenverbindung bzw. das Perfluoralkylsulfonat, die Base, die katalytische Menge metallischen Palladiums und der wasserlösliche Ligand in Wasser oder vorzugsweise in eine Mischung aus Wasser und einem oder mehreren inerten organischen Lösungsmitteln gegeben und bei einer Temperatur von 0°C bis 200°C, bevorzugt bei 30°C bis 170°C, besonders bevorzugt bei 50°C bis 150°C, insbesonders bevorzugt bei 60°C bis 120°C für einen Zeitraum von 1 h bis 100 h, bevorzugt 5 h bis 70 h, besonders bevorzugt 5 h bis 50 h, gerührt. Nach beendeter Umsetzung wird der als Feststoff anfallende Pd-Katalysator beispielsweise durch Filtration abgetrennt und das Rohprodukt vom Lösungsmittel bzw. den Lösungsmitteln befreit. Bei nicht völlig wasserlöslichen Produkten wird der Ligand bei der Trennung der Wasserphase vom Rohprodukt vollständig abgetrennt. Anschließend kann nach dem Fachmann bekannten und dem jeweiligen Produkt angemessenen Methoden, z.B. durch Umkristallisation, Destillation, Sublimation, Zonenschmelzen, Schmelzkristallisation oder Chromatographie, weiter aufgereinigt werden.

Das erfindungsgemäße Verfahren kann in einem Zweiphasensystem aus wäßriger Phase und fester Phase, d.h. dem Katalysator, durchgeführt werden. Die wäßrige Phase kann dabei neben Wasser auch ein wasserlösliches organisches Lösungsmittel enthalten.

Vorzugsweise wird jedoch in einem Dreiphasensystem aus einer wäßrigen Phase, einer organischen Phase und der festen Katalysator-Phase gearbeitet.

Das gilt insbesondere, wenn die eingesetzten Edukte oder die erfindungsgemäß hergestellten Produkte nicht vollständig wasserlöslich sind.

Für das erfindungsgemäße Verfahren geeignete organische Lösungsmittel sind beispielsweise Ether, z. B. Diethylether, Dimethoxymethan, Diethylenglykoldimethylether, Tetrahydrofuran, Dioxan, Diisopropylether, tert.-Butylmethylether, Kohlenwasserstöffe, z. B. Hexan, iso-Hexan, Heptan, Cyclohexan, Benzol, Toluol, Xylol, Alkohole, z. B. Methanol, Ethanol, 1-Propanol, 2-Propanol, Ethylenglykol, 1-Butanol, 2-Butanol, tert.-Butanol, Ketone, z. B. Aceton, Ethylmethylketon, iso-Butylmethylketon, Amide, z.B. Dimethylformamid, Dimethylacetamid, N-Methylpyrrolidon, Nitrile, z.B. Acetonitril, Propionitril, Butyronitril, und Mischungen derselben.

Bevorzugte organische Lösungsmittel sind Ether, wie Dimethoxyethan, Diethylenglykoldimethylether, Tetrahydrofuran, Dioxan, Diisopropylether, Kohlenwasserstoffe, wie Hexan, Heptan, Cyclohexan, Benzol, Toluol, Xylol, Alkohole, wie Methanol, Ethanol, 1-Propanol, 2-Propanol, 1-Butanol, 2-Butanol, tert.-Butanol, Ethylenglykol, Ketone, wie Ethylmethylketon, iso-Butylmethylketon, Amide, wie Dimethylformamid, Dimethylacetamid, N-Methylpyrrolidon, und Mischungen derselben.

Besonders bevorzugte Lösungsmittel sind Ether, z. B. Dimethoxyethan, Tetrahydrofuran, Kohlenwasserstoffe, z. B. Cyclohexan, Benzol, Toluol, Xylol, Alkohole, z. B. Ethanol, 1-Propanol, 2-Propanol, 1-Butanol, tert.-Butanol und Mischungen derselben.

In einer besonders bevorzugten Variante werden in dem erfindungsgemäßen Verfahren Wasser, ein oder mehrere in Wasser unlösliche und ein oder mehrere in Wasser lösliche Lösungsmittel eingesetzt.
Beispiele sind Mischungen aus Wasser, Toluol und Ethanol, Wasser, Toluol und Tetrahydrofuran und Wasser, Toluol und Acetonitril, vorzugsweise jeweils im Volumenverhältnis 1:2:1.

Basen, die bei dem erfindungsgemäßen Verfahren vorzugsweise Verwendung finden sind Alkali- und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate, sowie primäre, sekundäre und tertiäre Amine.

Besonders bevorzugt sind Alkali- und Erdalkalimetallhydroxide, Alkali- und Erdalkalimetallcarbonate und Alkalimetallhydrogencarbonate. Insbesondere bevorzugt sind Alkalimetallhydroxide, wie Natriumhydroxid und Kaliumhydroxid, sowie Alkalimetallcarbonate und Alkalimetallhydrogencarbonate, wie Lithiumcarbonat, Natriumcarbonat und Kaliumcarbonat.

Die Base wird bei dem erfindungsgemäßen Verfahren bevorzugt mit einem Anteil von 100 bis 1000 Mol-%, besonders bevorzugt 100 bis 500 Mol-%, ganz besonders bevorzugt 150 bis 400 Mol-%, insbesondere 180 bis 250 Mol-%, bezogen auf die aromatische Borverbindung, eingesetzt.

Als Katalysator dient Palladium in metallischer Form, im folgenden nur Palladium genannt, vorzugsweise Palladium in pulverisierter Form oder auf einem Trägermaterial, z.B. Palladium auf Aktivkohle, Palladium auf Aluminiumoxid, Palladium auf Bariumcarbonat, Palladium auf Bariumsulfat, Palladium auf Aluminiumsilikaten, wie Montmorillonit, Palladium auf SiO₂ und Palladium auf Calciumcarbonat, jeweils mit einem Palladiumgehalt von 0,5 bis 10 Gew.-%. Besonders bevorzugt sind Palladium in pulverisierter Form, Palladium auf Aktivkohle, Palladium auf Barium- und/oder Calciumcarbonat und Palladium auf Bariumsulfat, jeweils mit einem Palladiumgehalt von 0,5 bis 10 Gew.-%. Insbesondere bevorzugt ist Palladium auf Aktivkohle mit einem Palladiumgehalt von 5 oder 10 Gew.-%.
Es können auch Katalysatoren eingesetzt werden, die neben Palladium und dem Trägermaterial weitere Dotierstoffe, z.B. Blei (Lindlar-Katalysator), enthalten.

Der Palladiumkatalysator wird bei dem erfindungsgemäßen Verfahren mit einem Anteil von 0,01 bis 10 Mol-%, bevorzugt 0,05 bis 5 Mol-%, besonders bevorzugt 0,1 bis 3 Mol-%, insbesonders bevorzugt 0,1 bis 1,5 Mol-%, bezogen auf die aromatische Halogenverbindung oder das Perfluoralkylsulfonat, eingesetzt.

Für das erfindungsgemäße Verfahren geeignete wasserlösliche Liganden enthalten beispielsweise Sulfonsäuresalz- und/oder Sulfonsäurereste und/oder Carbonsäuresalz- und/oder Carbonsäurereste und/oder Phosphonsäuresalz und/oder Phosphonsäurereste und/oder Phosphoniumgruppen und/oder Peralkylammoniumgruppen und/oder Hydroxygruppen und/oder Polyethergruppen mit geeigneter Kettenlänge.

Bevorzugte Klassen von wasserlöslichen Liganden sind mit den obigen Gruppen substituierte Phosphane, wie Trialkylphosphane, Tricycloalkylphosphane, Triarylphosphane, Dialkylarylphosphane, Alkyldiarylphosphane und Heteroarylphosphane wie Tripyridylphosphan und Trifurylphosphan, wobei die drei Substituenten am Phosphor gleich oder verschieden, chiral oder achiral sein können und wobei einer oder mehrere der Liganden die Phosphorgruppen mehrerer Phosphane verknüpfen können und wobei ein Teil dieser Verknüpfung auch ein oder mehrere Metallatome sein können, Phosphite, Phosphinigsäureester und Phosphonigsäureester, Phosphole, Dibenzophosphole und Phosphoratome enthaltende cyclische bzw. oligo- und polycyclische Verbindungen.

Weitere geeignete Gruppen wasserlöslicher Komplexliganden umfassen beispielsweise Bipyridine, Phenanthroline, Porphyrine und Alizarine, die mit den obengenannten Gruppen modifiziert sind.

Bevorzugt eingesetzte wasserlösliche Phosphane sind solche der allgemeinen Formeln (I) bis (VII), 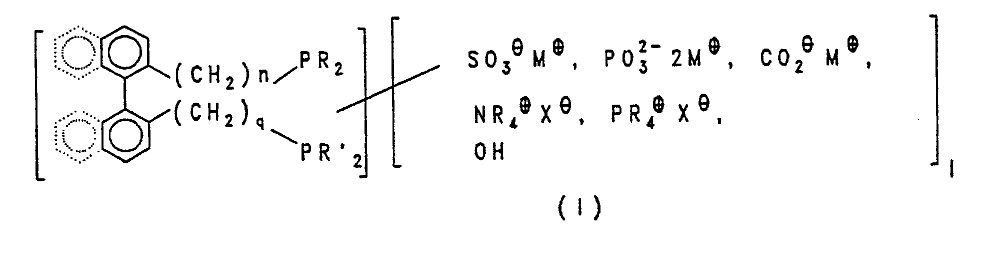 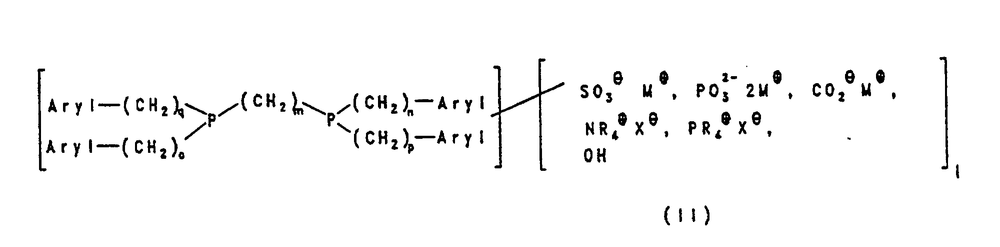 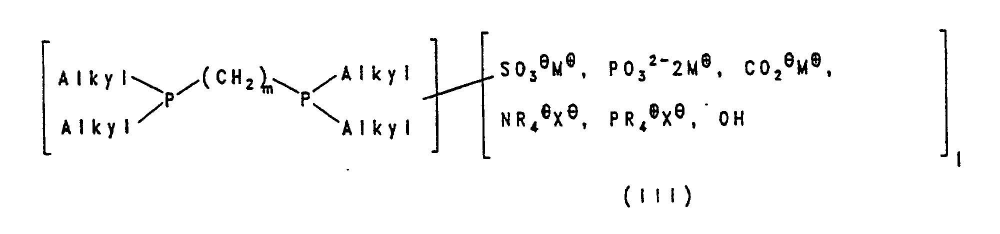 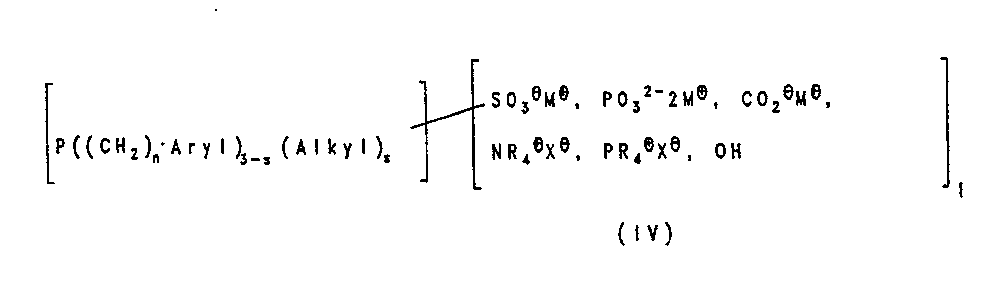 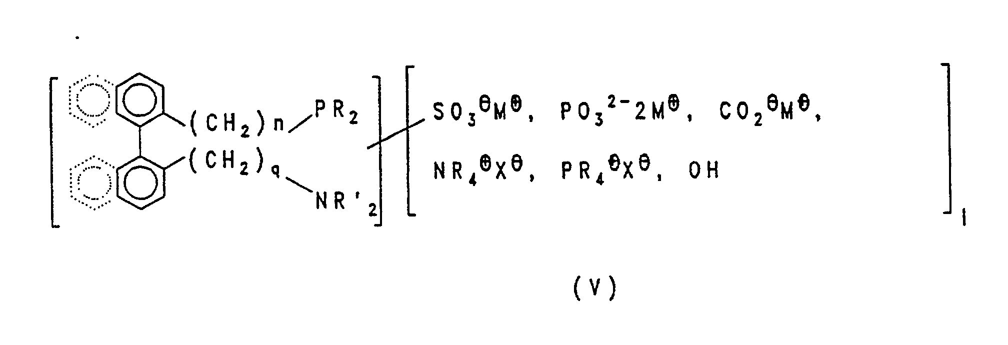 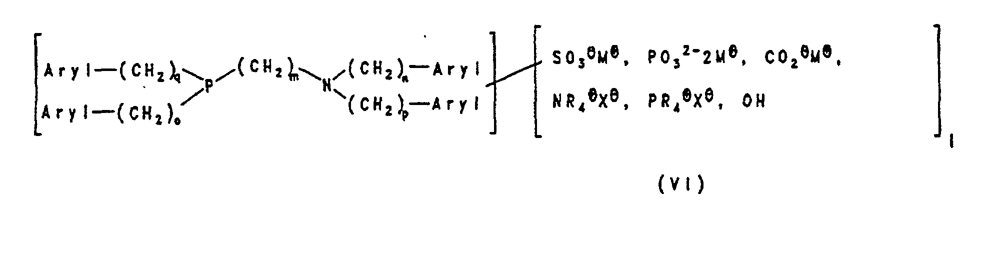 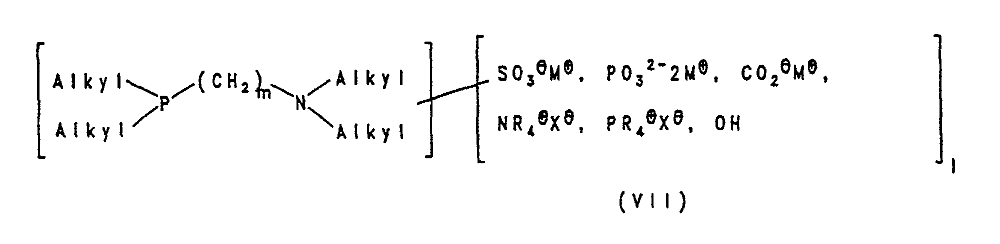 wobei die Symbole und Indizes folgende Bedeutungen haben:
- Aryl:: eine Phenyl- oder Naphthylgruppe, die auch einen oder mehrere Substituenten R tragen kann;
- Alkyl:: eine geradkettige oder verzweigte Alkylgruppe mit 1 bis 8 Kohlenstoffatomen;
- R,R':: Alkyl, Aryl oder Aralkyl mit 1 bis 18 Kohlenstoffatomen;
- M:: Alkali-, Erdalkalimetall oder NR₄;
- X:: Halogen, BF₄, OSO₂CF₃, 1/2[SO₄];
- l,m:: 1 bis 8;
- n,o,p,q:: 0, 1 bis 8;
- s:: 0, 1 bis 3.

Im folgenden sind Beispiele für besonders bevorzugte wasserlösliche Komplexliganden aufgeführt:
(R hat dabei, wenn nicht anders vermerkt, die in den Formeln (I) bis (VII) angegebene Bedeutung)
1. Sulfonierte Phosphane 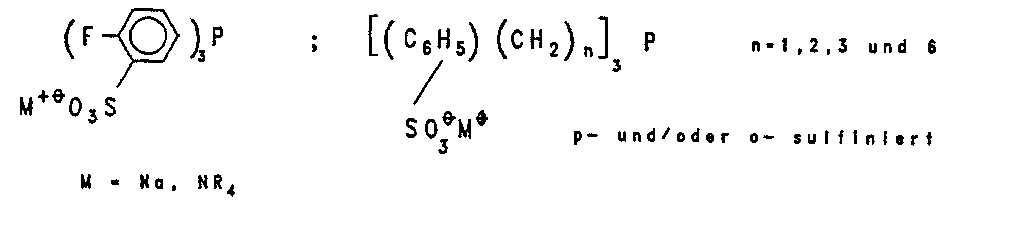 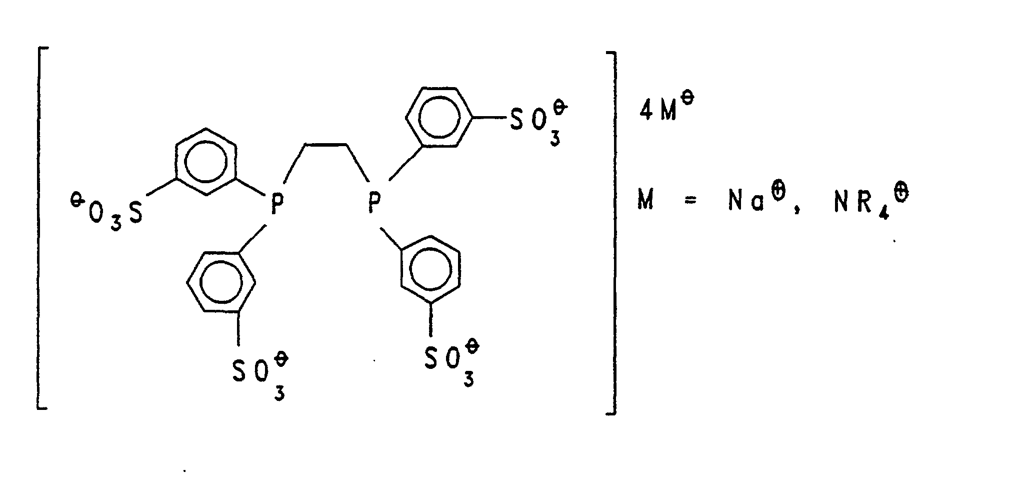 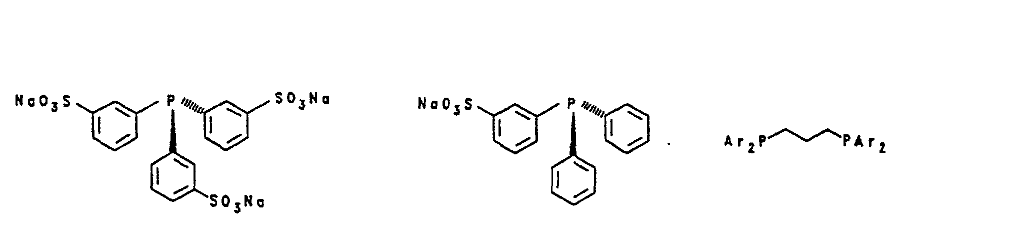 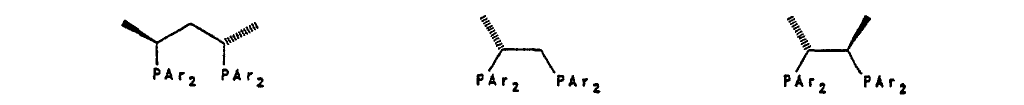 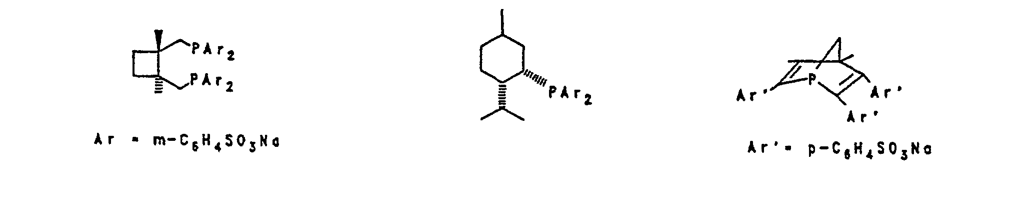 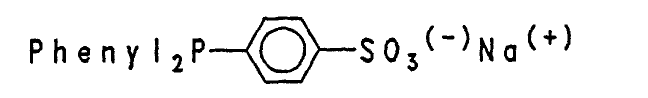 R₃₋ₙP(p-C₆H₄SO₃K)ₙ R = C₆H₅, 2-Pyridyl, 3-Pyridyl; n = 1-3 P[p-OC₆H₄SO₃(NH(i-octyl)₃]₃
1.1 Phosphane mit hydrophilen Gruppen in der Peripherie 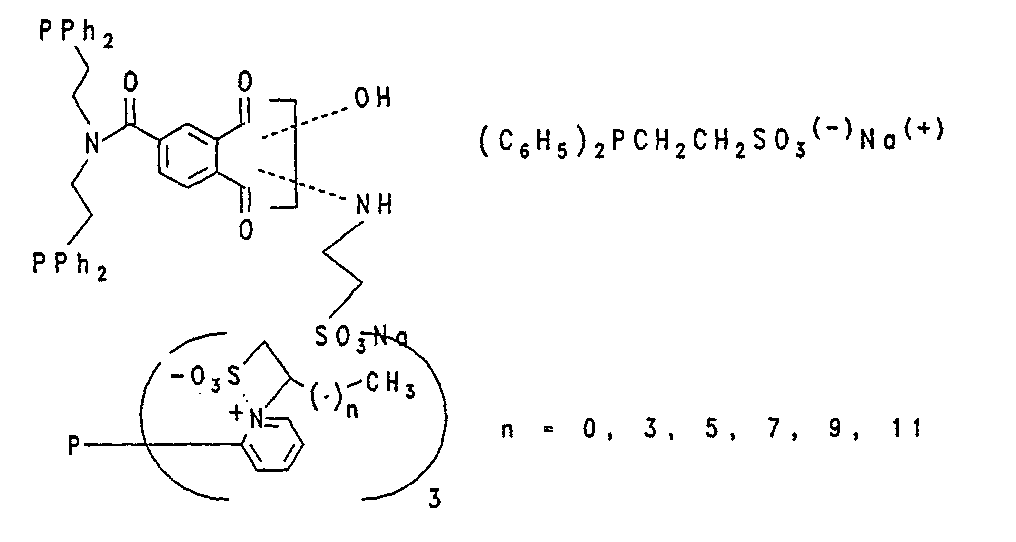
2. Phosphane mit quaternisierten Aminoalkyl- und Aminoaryl-Substituenten 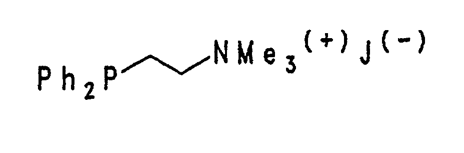 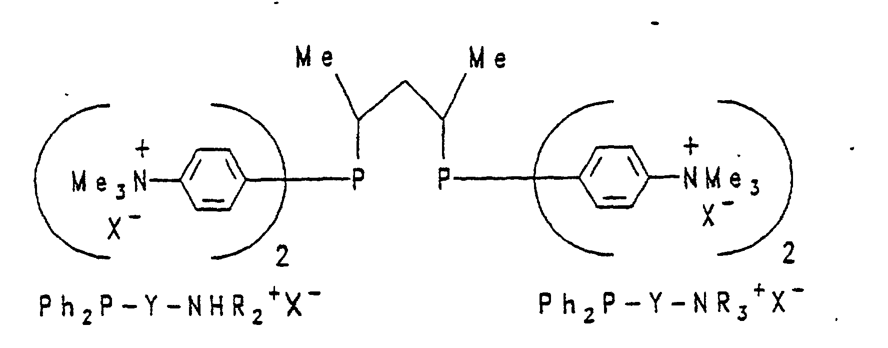 Y = -CH₂CH₂-, -CH(CH₃)CH₂-, -CH₂CH(CH₃)CH₂-; R =CH₃; X = J^{⊖}, Bu^{⊖}, Cl^{^{⊖}}, OSO₂CF₃^{^{⊖}}, BF₄^{^{⊖}}
3. Carboxylierte Phosphane 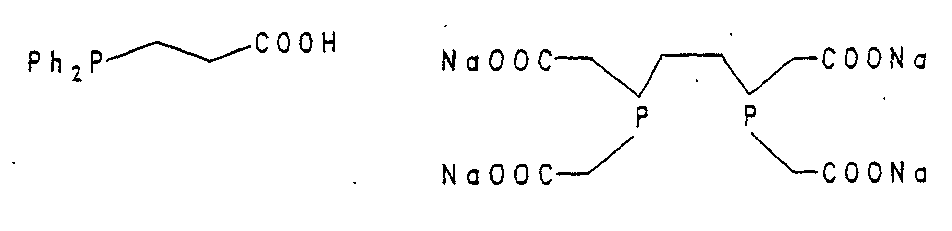 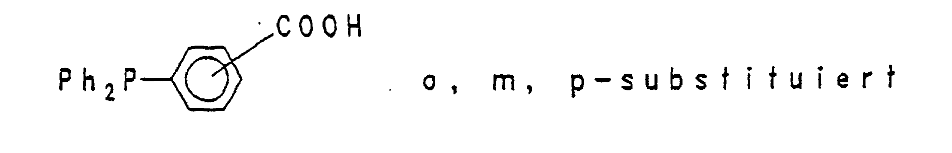 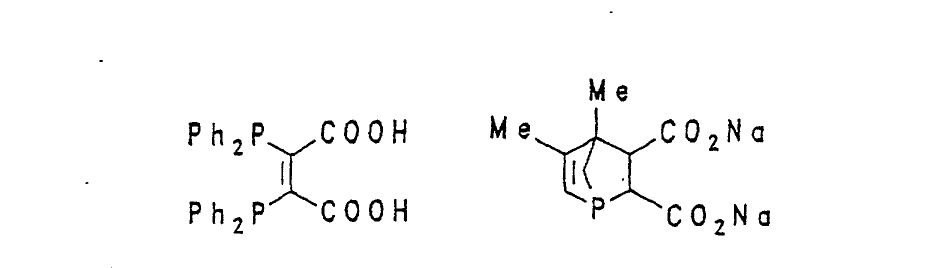
4. Phosphate mit Hydroxyalkyl- oder Polyether-Substituenten 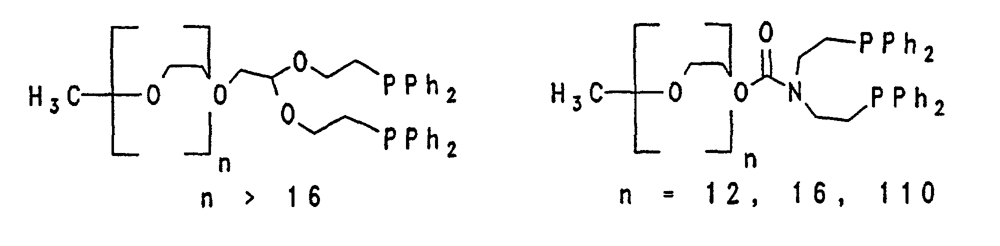 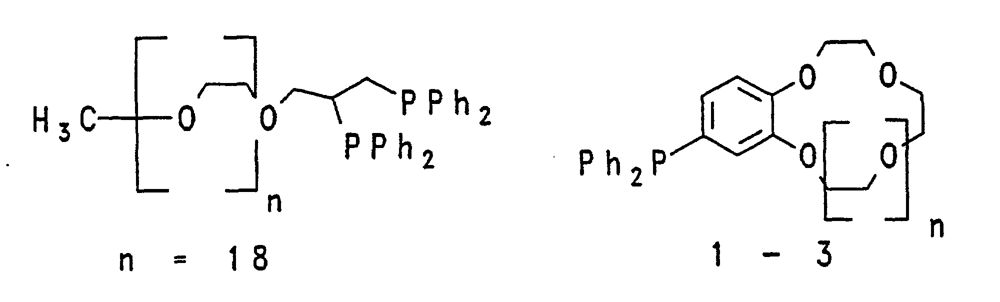
5. Phosphinoalkyl-phosphoniumsalze 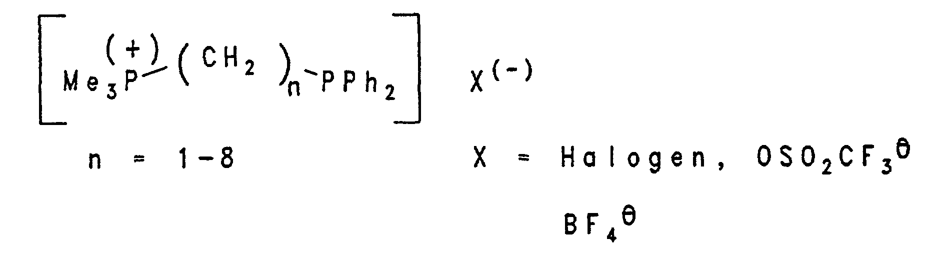
6. Phosphite

   P[-OC₆H₄SO₃{NH(i-Octyl)₃}]₃

   Insbesondere bevorzugte wasserlösliche Phosphinliganden sind: 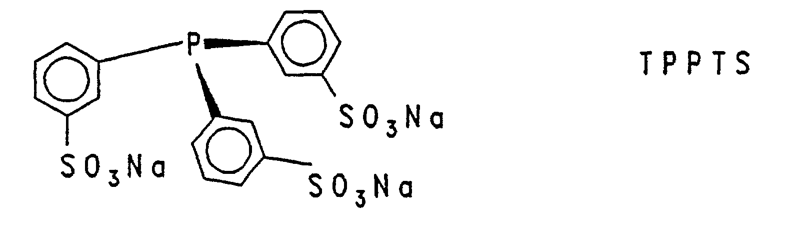 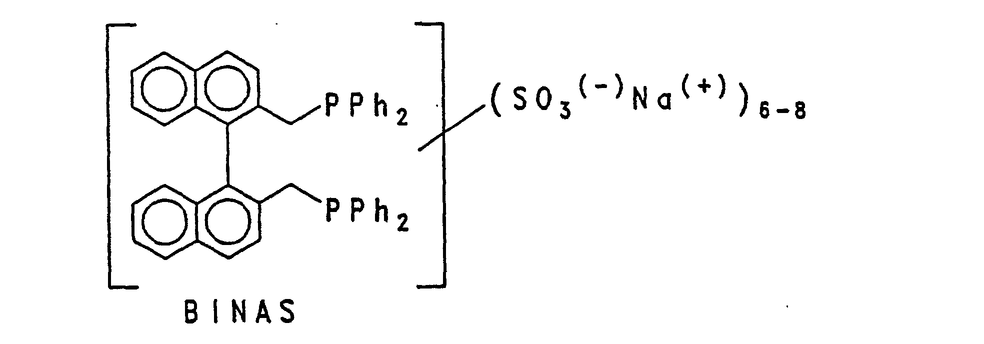 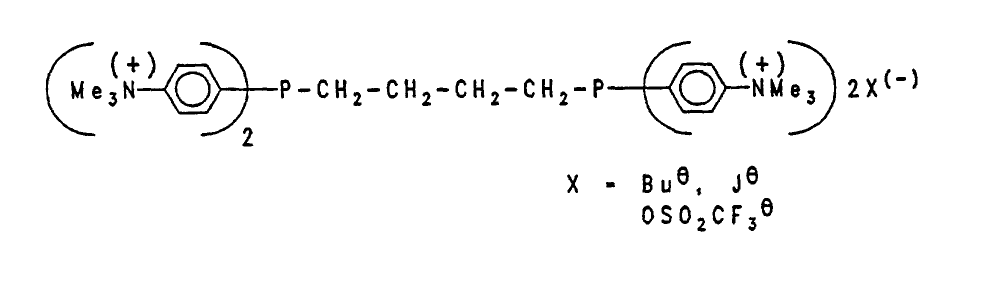 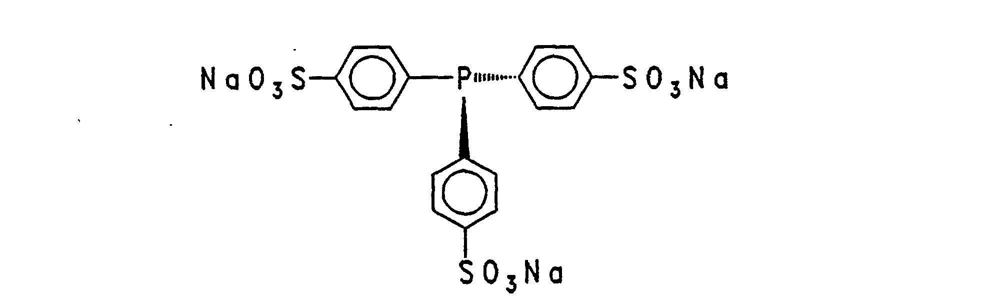
Der wasserlösliche Ligand wird bei dem erfindungsgemäßen Verfahren mit einem Anteil von 0,01 bis 20 Mol-%, bevorzugt 0,05 bis 15 Mol-%, besonders bevorzugt 0,05 bis 10 Mol-%, insbesonders bevorzugt 0,1 bis 6 Mol-%, bezogen auf die aromatische Halogenverbindung oder das Perfluoralkylsulfonat, eingesetzt.
Es können gegebenenfalls auch Mischungen zweier oder mehr verschiedener wasserlöslicher Liganden eingesetzt werden.
Die erfindungsgemäß einzusetzenden wasserlöslichen Komplexliganden sind zum großen Teil aus der Literatur bekannt. Die Synthesen sind beispielsweise beschrieben in W.A. Herrmann und C.W. Kohlpainter, Angew. Chem. Int. Ed.
Engl. 32 (1993) 1524 und der dort zitierten Literatur oder können nach literaturbekannten, dem Fachmann geläufigen Methoden erfolgen. Die Herstellung von BINAS ist beispielsweise in der deutschen Patentanmeldung P 42 44 274 beschrieben.

Ausgangsverbindungen für das erfindungsgemäße Verfahren sind zum einen aromatische Borverbindungen der Formel (VIII),

Aryl ― BQ₁Q₂ (VIII)

worin
- Aryl: ein aromatischer Rest ist und
- Q₁, Q₂: gleich oder verschieden -OH, C₁-C₄-Alkoxy, C₁-C₄-Alkyl, Phenyl, das gegebenenfalls durch C₁-C₄-Alkyl, C₁-C₄-Alkoxy oder Halogen substituiert sein kann, oder Halogen bedeuten oder Q₁ und Q₂ zusammen bilden eine C₁-C₄-Alkylendioxy-Gruppe, eine Methylengruppe, die gegebenenfalls durch eine oder zwei C₁-C₄-Alkylgruppen substituiert sein kann, oder Q₁ und Q₂ und das Boratom zusammen sind Teil einer Boroxinrings der Formel (IX):

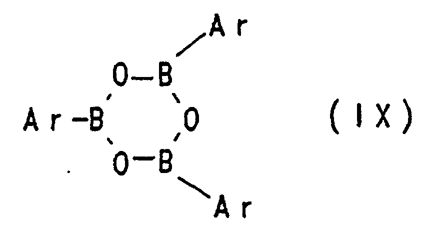

Bevorzugt sind aromatische Borverbindungen der Formel (X),

R¹(-A¹)ₖ(-M¹)ₗ-A²-B Q₁Q₂ (X)

wobei R¹, A¹, A², M¹, Q₁, Q₂, k und l die folgende Bedeutung haben:

R¹ ist Benzyloxy, H, F, Cl, Br, -NC, -CN, -CF₃, -OCF₃ oder ein geradkettiger oder verzweigter (mit oder ohne asymmetrisches C-Atom) Alkylrest mit 1 bis 18 C-Atomen, wobei auch eine oder zwei nicht benachbarte -CH₂-Gruppen durch -O-, -S-, -CO-, -CO-O-, -O-CO-, -CO-S-, -S-CO-, -O-CO-O-, -CH=CH-, -C≡C-, -SO₂-, -CON(H, C₁-C₈-Alkyl)-, 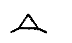 oder -Si(CH₃)₂- ersetzt sein können, und wobei auch ein oder mehrere H-Atome des Alkylrestes durch F, Cl, Br oder CN substituiert sein können;

A¹ ist 1,4-Phenylen, Pyrazin-2,5-diyl, Pyridazin-3,6-diyl, Pyridin-2,5-diyl, Pyrimidin-2,5-diyl, trans-1,4-Cyclohexylen, Naphthalin-2,6-diyl, wobei ein oder mehrere H Atome gleich oder verschieden durch Substituenten L ersetzt sein können, wobei L die unter R¹ genannten Bedeutungen hat, oder 4,4-Dimethylisoxazolin ist, und wobei eine oder zwei nicht benachbarte CH₂-Gruppen des Cyclohexylens durch -O- oder -S- ersetzt sein können (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Bicyclo[2.2.2]octan-1,4-diyl, 1,3-Dioxaborinan-2,5-diyl oder trans-Dekalin-2,6-diyl;

A² ist 1,4-Phenylen, Pyrazin-2,5-diyl, Pyridazin-3,6-diyl, Pyridin-2,5-diyl, Pyrimidin-2,5-diyl, Naphthalin-2,6-diyl, wobei ein oder mehrere H Atome gleich oder verschieden durch Substituenten L ersetzt sein können, wobei L die unter R¹ genannten Bedeutungen hat, oder 4,4-Dimethylisoxazolin ist, 1,3,4-Thiadiazol-2,5-diyl, 1,3-Thiazol-2,4-diyl, 1,3-Thiazol-2,5-diyl, Thiophen-2,4-diyl oder Thiophen-2,5-diyl;

M¹ ist
-O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-, -CH₂CH₂CH₂-O-, -OCH₂CH₂CH₂-, -CH₂CH₂CO-O-, -O-COCH₂CH₂-, und
- Q₁, Q₂: sind gleich oder verschieden -OH, C₁-C₄-Alkoxy oder Halogen oder Q₁ und Q₂ zusammen bilden eine C₁-C₄-Alkylendioxy-Gruppe oder Q₁ und Q₂ und das Boratom zusammen sind teil eines Boroxinrings der Formel (IX): 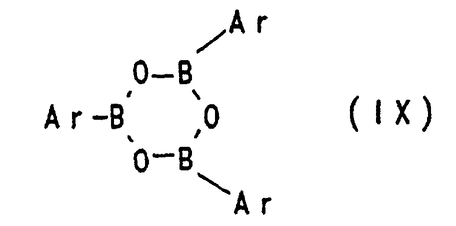
k, l bedeuten jeweils unabhängig voneinander Null oder Eins.

Bevorzugt bedeutet R¹, Benzyloxy, H, F, Cl, -CF₃, OCF₃, CN oder ein geradkettiger oder verzweigter (mit oder ohne asymmetrisches C-Atom) Alkylrest mit 1 bis 18 C-Atomen, wobei auch eine oder zwei nicht benachbarte -CH₂-Gruppen durch -O-, -CO-, -CO-O-, -OCO-, -O-CO-O-, -CH=CH-, -C≡C-, 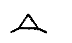 oder -Si(CH₃)₂- ersetzt sein können, und wobei auch ein oder mehrere H-Atome des Alkylrests durch F, Cl oder CN substituiert sein können.

Besonders bevorzugt bedeutet R¹ Benzyloxy, H oder ein geradkettiger oder verzweigter (mit oder ohne asymmetrisches C-Atom) Alkylrest mit 1 bis 18 C-Atomen, wobei auch eine oder zwei nicht benachbarte -CH₂-Gruppen durch -O-, -CH=CH-, -C≡C-, 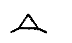 oder -Si(CH₃)₂- ersetzt sein können.

Bevorzugt bedeutet A¹ 1,4-Phenylen, Pyrazin-2,5-diyl, Pyridazin-3,6-diyl, Naphthalin-2,6-diyl, Pyridin-2,5-diyl, Pyrimidin-2,5-diyl, wobei ein oder mehrere H-Atome gleich oder verschieden durch Substituenten L ersetzt sein können, wobei L die unter R¹ genannten Bedeutungen hat, trans-1,4-Cyclohexylen, worin eine oder zwei nicht benachbarte -CH₂-Gruppen durch -O- ersetzt sein können, (1,3,4)-Thiadiazol-2,5-diyl, oder Bicyclo[2.2.2]octan-1,4-diyl.

Besonders bevorzugt bedeutet A¹ 1,4-Phenylen, 2-Fluor-1,4-Phenylen, 2,3-Difluor-1,4-Phenylen, 2,6-Difluor-1,4-Phenylen, 2,5-Difluor-1,4-Phenylen, sowie Pyridin-2,5-diyl, Pyrimidin-2,5-diyl, wobei ein oder zwei H-Atome gleich oder verschieden durch Substituenten L ersetzt sein können, wobei L die unter R¹ genannten Bedeutungen hat, trans-1,4-Cyclohexylen.

Bevorzugt bedeutet A² 1,4-Phenylen, Pyrazin-2,5-diyl, Pyridazin-3,6-diyl, Pyridin-2,5-diyl, Pyrimidin-2,5-diyl, Naphthalin-2,6-diyl, wobei ein oder mehrere H-Atome gleich oder verschieden durch Substituenten L ersetzt sein können, wobei L die unter R¹ genannten Bedeutungen hat.

Besonders bevorzugt bedeutet A² 1,4-Phenylen, 2-Fluor-1,4-Phenylen, 2,3-Difluor-1,4-phenylen, 2,6-Difluor-1,4-Phenylen, 2,5-Difluor-1,4-phenylen, sowie Pyridin-2,5-diyl, oder Naphthalin-2,6-diyl, wobei ein oder mehrere H-Atome gleich oder verschieden durch Substituenten L ersetzt sein können, wobei L die unter R¹ genannten Bedeutungen hat.

Bevorzugt bedeutet M¹ -O-, -CO-, -CO-O-, -O-CO-, -O-CO-O-, -CH₂-O-, -O-CH₂-, -CH₂-CH₂-, -CH=CH-, -C≡C-, -CH₂CH₂CH₂-O-, -O-CH₂CH₂CH₂-, -CH₂CH₂CO-O- oder -O-CO-CH₂CH₂-.

Besonders bevorzugt bedeutet M¹ -O-, -CH₂-O-, -O-CH₂-, -CH₂CH₂-, -CH = CH-oder -C≡C-.

Insbesondere bevorzugt sind nachfolgend aufgeführte aromatische Boronsäuren der Formel Xa bis Xh: 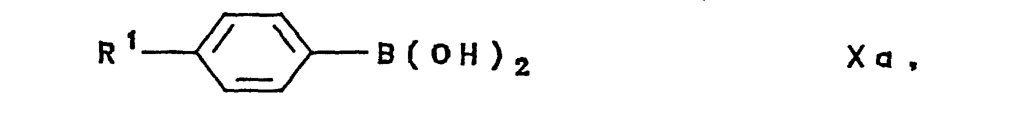 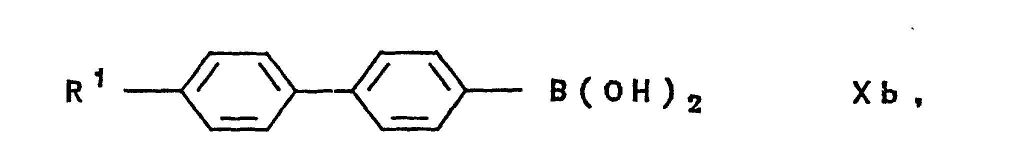 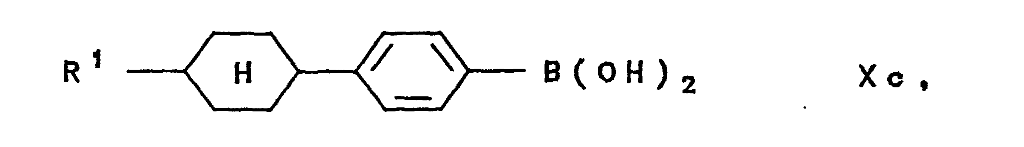 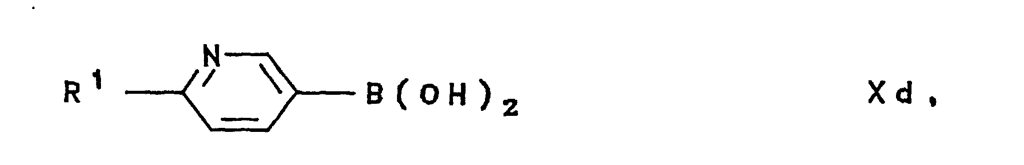 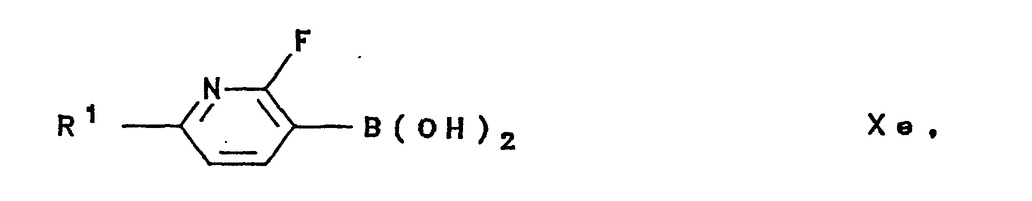 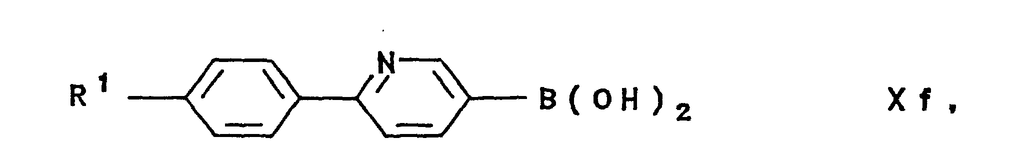 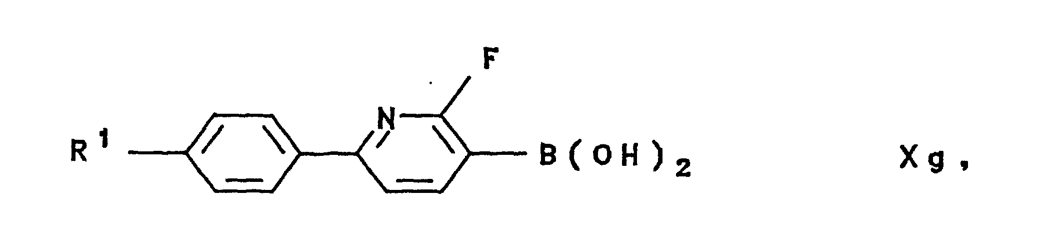 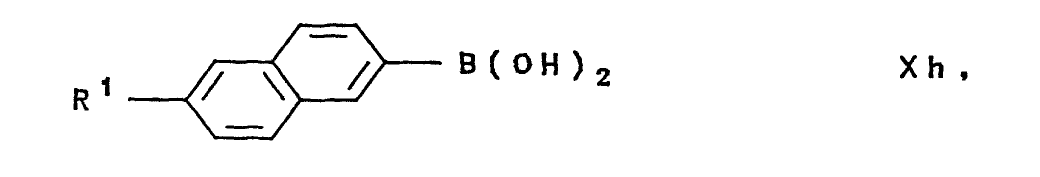 wobei R¹, Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und Pentadecyl, sowie Methoxy, Ethoxy, Propoxy, Butoxy, Pentoxy, Hexoxy, Heptoxy, Octoxy, Nonoxy, Decoxy, Undecoxy, Dodecoxy, Tridecoxy, Tetradecoxy und Pentadecoxy bedeuten.

Die verwendeten aromatischen Borverbindungen sind entweder bekannt oder können nach bekannten Methoden, wie beispielsweise in Houben Weyl Methoden der Organischen Chemie, Georg Thieme-Verlag, Stuttgart, Band 13/3a beschrieben, hergestellt werden. So ist es beispielsweise möglich, aus aromatischen Alkalimetall- und Magnesiumverbindungen durch Umsetzung mit Trialkoxyboranen und anschließender Hydrolyse Boronsäuren, vorzugsweise solche der Formel II, zu erhalten.

Die zweite Klasse von Ausgangsverbindungen für das erfindungsgemäße Verfahren sind aromatische Halogenverbindungen oder aromatische Perfluoralkylsulfonate, vorzugsweise solche der Formel XI,

X-A³(-M²)ₘ(-A⁴)ₙ-R² (XI),

wobei R², A³, A⁴, M², X m und n die folgende Bedeutung haben.

R² ist Benzyloxy, H, F, Cl, Br, -NC, -CN, -CF₃, -OCF₃, Isoxazolin oder ein geradkettiger oder verzweigter (mit oder ohne asymmetrisches C-Atom) Alkylrest mit 1 bis 18 C-Atomen, wobei auch eine oder zwei nicht benachbarte -CH₂-Gruppen durch -O-, -S-, -CO-, -CO-O-, -O-CO-, -CO-S-, -S-CO-, -O-CO-O-, -SO₂-, CON(H,C₁-C₈-Alkyl), -CH=CH-, -C≡C-, 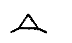 oder -Si(CH₃)₂- ersetzt sein können, und wobei auch ein oder mehrere H-Atome des Alkylrestes durch F, Cl, Br oder CN substituiert sein können,

A⁴ ist 1,4-Phenylen, Pyrazin-2,5-diyl, Pyridazin-3,6-diyl, Pyridin-2,5-diyl, Pyrimidin-2,5-diyl, Naphthalin-2,6-diyl, trans-1,4-Cyclohexylen, worin eine oder zwei nicht benachbarte CH₂-Gruppen durch -O- oder -S- ersetzt sein können und wobei ein oder mehrere H-Atome gleich oder verschieden durch Substituenten L ersetzt sein können, wobei L die unter R¹ genannten Bedeutungen hat, oder CHO, oder 4,4-Dimethylisoxazolin ist, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Bicyclo[2.2.2]octan-1,4-diyl, 1,3-Dioxaborinan-2,5-diyl oder trans-Dekalin-2,6-diyl,

A³ ist 1,4-Phenylen, Pyrazin-2,5-diyl, Pyridazin-3,6-diyl, Pyridin-2,5-diyl, Pyrimidin-2,5-diyl, Naphthalin-2,6-diyl, wobei ein oder mehrere H-Atome gleich oder verschieden durch Substituenten L ersetzt sein können, wobei L die unter R¹ genannten Bedeutungen hat, oder CHO, oder 4,4-Dimethylisoxazolin ist, 1,3,4-Thiadiazol-2,5-diyl, 1,3-Thiazol-2,4-diyl, 1,3-Thiazol-2,5-diyl, Thiophen-2,4-diyl oder Thiophen-2,5-diyl,

M² ist
-O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-,
- CH₂CH₂CH₂-O-, -OCH₂CH₂CH₂-, -CH₂CH₂CO-O-, -O-COCH₂CH₂-, und
   - X: ist Chlor, Brom, lod oder -OSO₂-CₚF₂ₚ₊₁, worin p einen ganzzahligen Wert von 1 bis 10 darstellt;
m, n bedeuten jeweils unabhängig voneinander Null oder Eins.

Bevorzugt bedeutet R² Benzyloxy, H, F, Cl, Br, -CN, -CF₃, -OCF₃ oder ein geradkettiger oder verzweigter (mit oder ohne asymmetrisches C-Atom) Alkylrest mit 1 bis 18 C-Atomen, wobei auch eine oder zwei nicht benachbarte -CH₂-Gruppen durch -O-, -CO-, -CO-O-, -O-CO-, -O-CO-O-, -CH=CH-, -C≡C-, 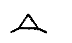 oder -Si(CH₃)₂- ersetzt sein können, und wobei auch ein oder mehrere H-Atome des Alkylrestes durch F, Cl oder CN substituiert sein können.

Besonders bevorzugt bedeutet R² Benzyloxy, H, Cl, Br oder ein geradkettiger oder verzweigter (mit oder ohne assymmetrisches C-Atom) Alkylrest mit 1 bis 18 C-Atomen, wobei auch eine oder zwei nicht benachtbarte -CH₂-Gruppen durch -O-, -CO-, -CO-O-, -O-, -O-CO-, -O-CO-O-, -CH=CH-, -C≡C-, 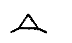 oder -Si(CH₃)-ersetzt sein können.

Bevorzugt bedeutet A³ 1,4-Phenylen, Pyrazin-2,5-diyl, Pyridazin-3,6-diyl, Pyridin-2,5-diyl, Pyrimidin-2,5-diyl, Naphthalin-2,6-diyl, wobei ein oder mehrere H-Atome gleich oder verschieden durch Substituenten L ersetzt sein können, wobei L die unter R¹ genannten Bedeutungen hat, CHO oder 4,4-Dimethylisoxazolin ist oder 1,3,4-Thiadiazol-2,5-diyl.

Besonders bevorzugt bedeutet A³ 1,4-Phenylen, 2-Fluor-1,4-phenylen, 2,3-Difluor-1,4-phenylen, 2,6-Difluor-1,4-phenylen, 2,5-Difluor-1,4-phenylen, sowie Pyrazin-2,5-diyl, Pyridazin-2,5-diyl, Pyrimidin-2,5-diyl oder Naphthalin-2,6-diyl, wobei ein oder mehrere H-Atome gleich oder verschieden durch Substituenten L ersetzt sein können, wobei L die unter R¹ genannten Bedeutungen hat, CHO oder 4,4-Dimethylisoxazolin ist.

Bevorzugt bedeutet A⁴ 1,4-Phenylen, Pyrazin-2,5-diyl, Pyridazin-3,6-diyl, Pyridin-2,5-diyl, Pyrimidin-2,5-diyl, trans-1,4-Cyclohexylen, worin eine oder zwei nicht benachbarte CH₂-Gruppen durch -O- ersetzt sein können, Naphthalin-2,6-diyl, wobei ein oder mehrere H-Atome gleich oder verschieden durch Substituenten L ersetzt sein können, wobei L die unter R¹ genannten Bedeutungen hat, (1,3,4)-Thiadiazol-2,5-diyl oder Bicyclo[2.2.2]octan-1,4-diyl.

Besonders bevorzugt bedeutet A⁴ 1,4-Phenylen, 2-Fluor-1,4-phenylen, 2,3-Difluor-1,4-phenylen, 2,6-Difluor-1,4-phenylen, 2,5-Difluor-1,4-phenylen, sowie Pyridin-2,5-diyl, Pyrimidin-2,5-diyl, trans-1,4-Cyclohexylen, Naphthalin-2,6-diyl, wobei ein oder mehrere H-Atome gleich oder verschieden durch Substituenten L ersetzt sein können, wobei L die unter R¹ genannten Bedeutungen hat.

Bevorzugt bedeutet M² -O-, -CO-, -CO-O-, -O-CO-, -O-CO-O-, -CH₂-O-, -O-CH₂-, -CH₂CH₂-, -CH=CH-, -C≡C-, -CH₂CH₂CH₂-O-, -O-CH₂CH₂CH₂-, -CH₂CH₂CO-O- oder -O-CO-CH₂CH₂-.

Besonders bevorzugt bedeutet M² -O-, -CO-, -CO-O-, -O-CO-, -O-CO-O-, -CH₂-O-, -O-CH₂-, -CH₂CH₂-, -CH=CH-, -C≡C-, -CH₂CH₂CH₂-O-, -O-CH₂CH₂CH₂-, -CH₂CH₂CO-O- oder -O-CO-CH₂CH₂.

Bevorzugt bedeutet X Brom, lod oder OSO₂-CₚF₂ₚ₊₁, worin p einen ganzzahligen Wert von 1 bis 10 darstellt. Besonders bevorzugt bedeutet X Brom.

Insbesondere bevorzugt sind die nachfolgend aufgeführten aromatischen Halogenverbindungen der Formel XI 1 bis XI 24, 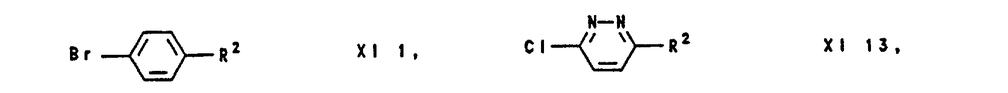 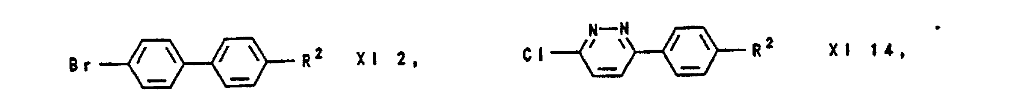 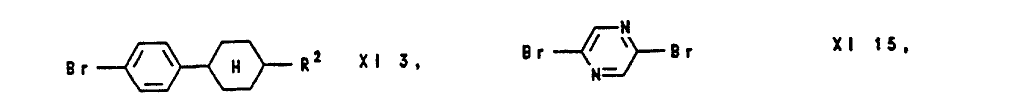 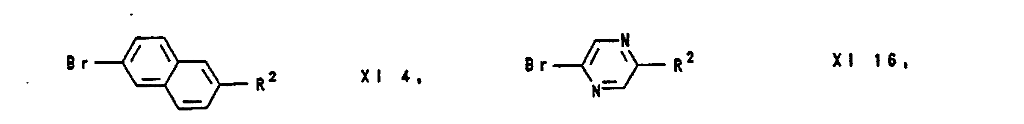 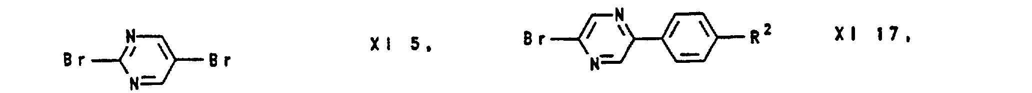 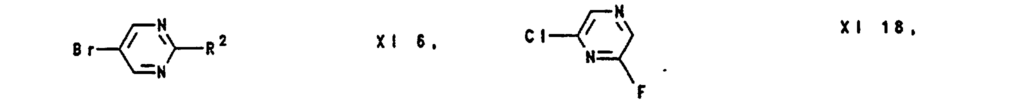 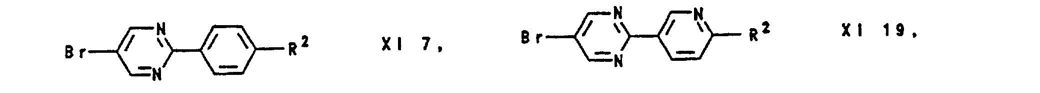 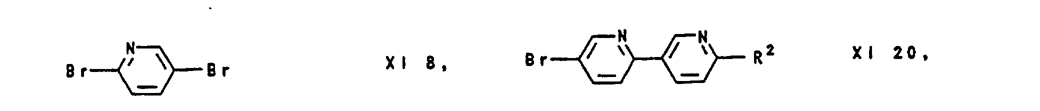 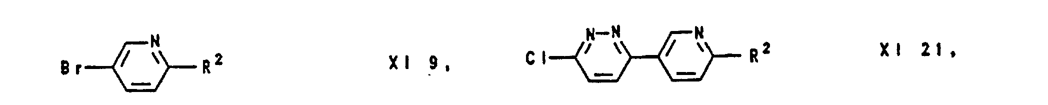 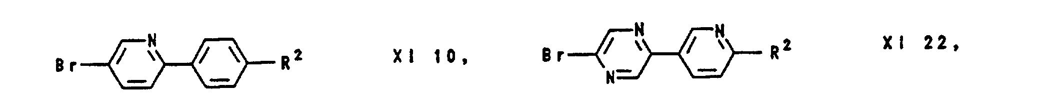 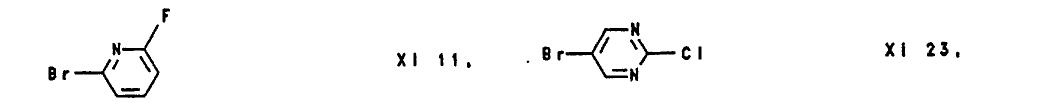 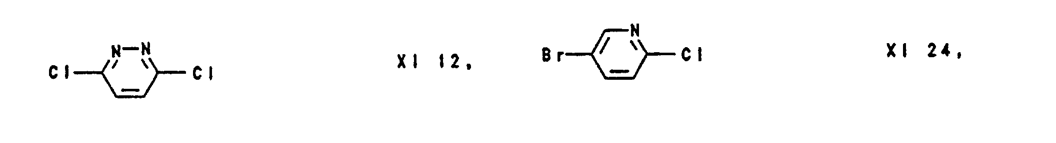 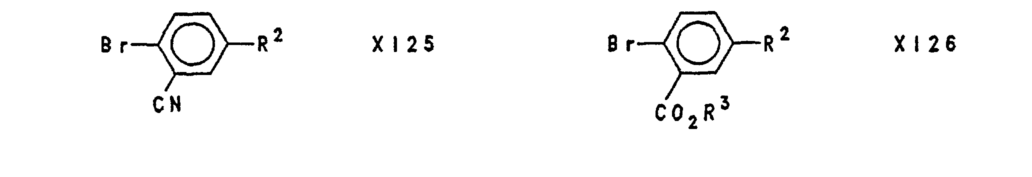 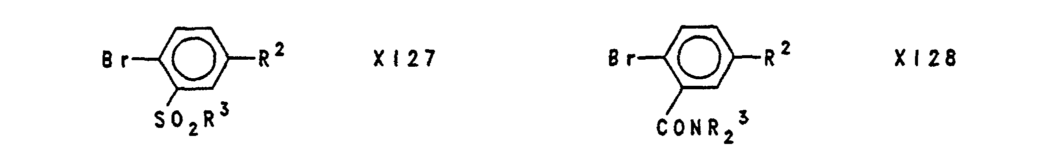 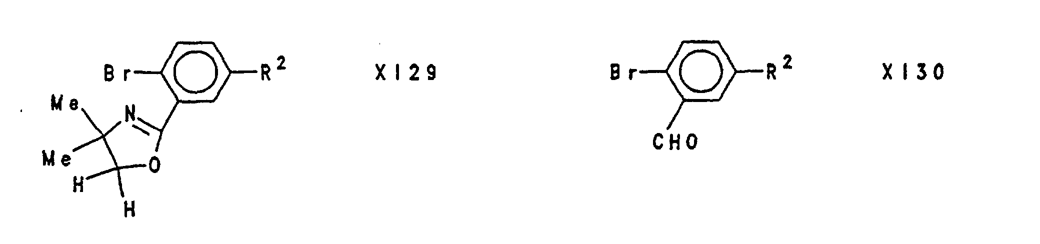 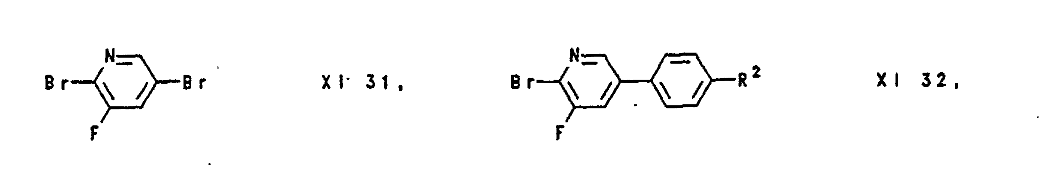 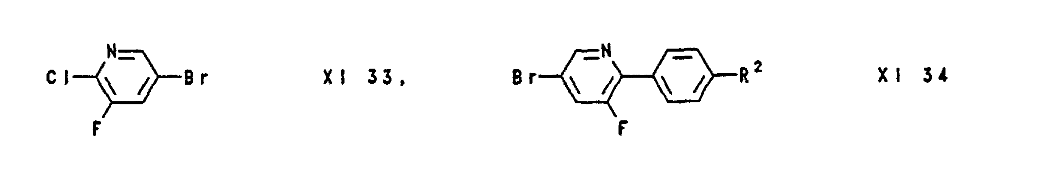 wobei R² und R³ Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und Pentadecyl, sowie Methyloxy, Ethyloxy, Propyloxy, Butyloxy, Pentyloxy, Hexyloxy, Heptyloxy, Octyloxy, Nonyloxy, Decyloxy, Undecyloxy, Dodecyloxy, Tridecyloxy, Tetradecyloxy und Pentadecyloxy bedeuten.

Die verwendeten aromatischen Halogenverbindungen und Perfluoralkylsulfonate sind entweder bekannt oder können nach bekannten Methoden, wie beispielsweise in Houben Weyl, Methoden der Organischen Chemie, Georg Thieme Verlag, Stuttgart, Band 5/3 und 5/4 beschrieben, hergestellt werden. Beispielsweise lassen sich aromatische Halogenide dadurch erhalten, daß man in einem entsprechenden Diazoniumsalz die Diazoniumgruppe durch Chlor, Brom oder lod ersetzt.
Desweiteren lassen sich Hydroxy-Stickstoffheterocyclen mit Hilfe von Phosphortrihalogeniden und Phosphoroxytrihalogeniden in die entsprechenden Halogenide überführen.
Das erfindungsgemäße Verfahren zur Kreuzkupplung von aromatischen Borverbindungen mit aromatischen Halogenverbindungen oder Perfluoralkylsulfonaten kann ebenfalls zur Herstellung von Verbindungen der Formel XI benutzt werden. Perfluoralkylsulfonate der Formel XI, worin X OSO₂-CₙF₂ₙ₊₁ bedeutet, können durch Veresterung entsprechender Alkohol der Formel XI, worin X eine Hydroxylgruppe bedeutet, mit Perfluoralkansulfonsäuren oder ihren reaktiven Derivaten hergestellt werden. Die entsprechenden Perfluoralkansulfonsäuren sind bekannt. Als reaktionsfähige Derivate der genannten Perfluoralkansulfonsäuren eignen sich insbesondere die Säurehalogenide, vor allem die Chloride und Bromide, ferner die Anhydride.

Produkte des erfindungsgemäßen Verfahrens sind mehrkernige aromatische Verbindungen.

Als bevorzugte Produkte des erfindungsgemäßen Verfahrens fallen Verbindungen der allgemeinen Formel XII an,

R¹(-A¹)ₖ(-M¹)ₗ-A²-A³(-M²)ₘ(-A⁴)ₙ-R² (XII)

wobei R¹ und R² unabhänigig voneinander Benzyloxy, H, F, Cl, Br, -NC, -CN, -CF₃, -OCF₃ oder ein geradkettiger oder verzweigter (mit oder ohne asymmetrisches C-Atom) Alkylrest mit 1 bis 18 C-Atomen sein können, wobei auch eine oder zwei nicht benachbarte -CH₂-Gruppen durch -O-, -S-, -CO-, -CO-O-, -O-CO-, -CO-S-, -S-CO-, -O-CO-O-, -CH=CH-, -C≡C-, -SO₂-, CON(H,C₁-C₈-Alkyl), 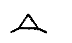 oder -Si(CH₃)₂- ersetzt sein können, und wobei auch ein oder mehrere H-Atome des Alkylrestes durch F, Cl, Br oder CN substituiert sein können,

A¹ und A⁴ jeweils unabhängig voneinander 1,4-Phenylen, Pyrazin-2,5-diyl, Pyridazin-3,6-diyl, Pyridin-2,5-diyl, Pyrimidin-2,5-diyl, Naphthalin-2,6-diyl, trans-1,4-Cyclohexylen, worin eine oder zwei nicht benachbarte CH₂-Gruppen durch -O- oder -S- ersetzt sein können und wobei ein oder mehrere H-Atome gleich oder verschieden durch Substituenten L ersetzt sein können, wobei L die unter R¹ genannten Bedeutungen hat oder 4,4-Dimethylisoxazolin ist, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Bicyclo[2.2.2]octan-1,4-diyl, 1,3-Dioxaborinan-2,5-diyl oder trans-Dekalin-2,6-diyl,

A² und A³ jeweils unabhänig voneinander 1,4-Phenylen, Pyrazin-2,5-diyl, Pyridazin-3,6-diyl, Pyridin-2,5-diyl, Pyrimidin-2,5-diyl, Naphthalin-2,6-diyl, wobei ein oder mehrere H-Atome gleich oder verschieden durch Substituenten L ersetzt sein können, wobei L die unter R¹ genannten Bedeutungen hat, 4,4-Dimethylisoxazolin oder im Falle von A³ auch CHO ist, 1,3,4-Thiadiazol-2,5-diyl, 1 ,3-Thiazol-2,4-diyl, 1,3-Thiazol-2,5-diyl, Thiophen-2,4-diyl oder Thiophen-2,5-diyl sein können,

M¹ und M² jeweils unabhänig voneinander
-O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-, -CH₂CH₂CH₂-O-, -OCH₂CH₂CH₂-, -CH₂CH₂CO-O-, -O-COCH₂CH₂-sein können,
und k, l, m, n jeweils unabhängig voneinander Null oder Eins bedeuten.

Bevorzugte und besonders bevorzugte Varianten von R¹, R², A¹, A², A³, A⁴, M¹, M², k, l, m, n sind die in den Formeln II und III angegebenen.

Insbesondere bevorzugt sind die nachfolgend aufgeführten Verbindungen der Formel XII 1 bis XII 94 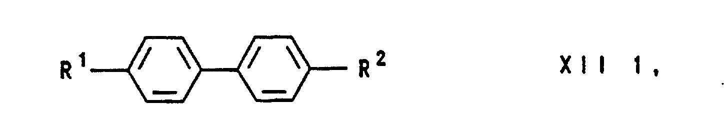 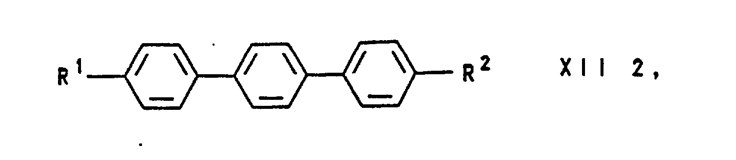 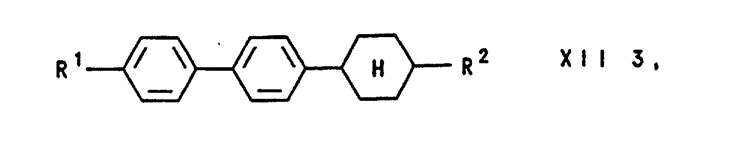 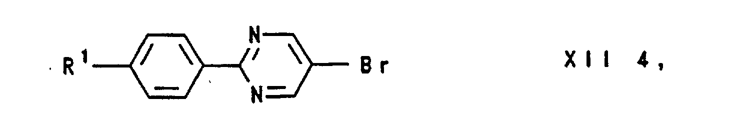 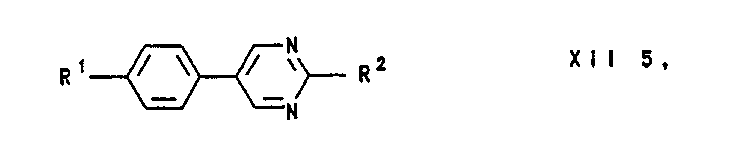 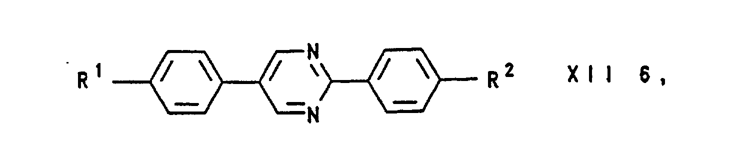 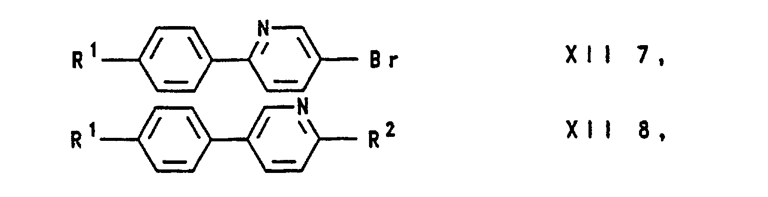 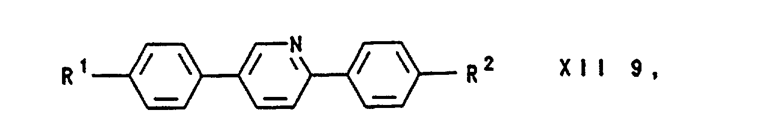 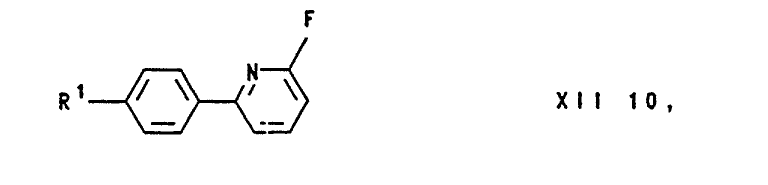 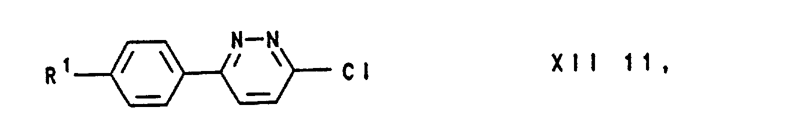 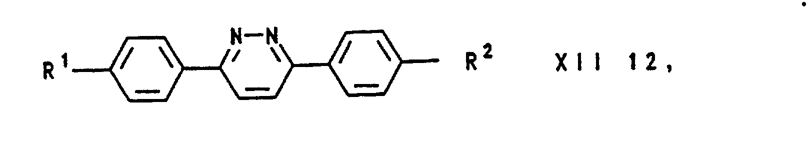 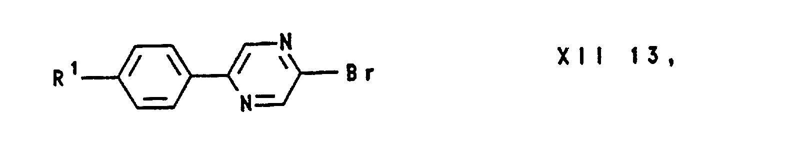 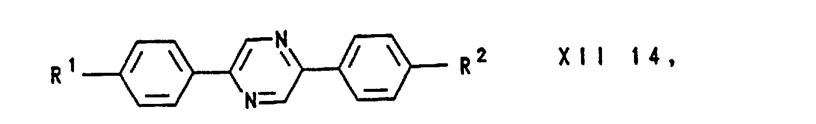 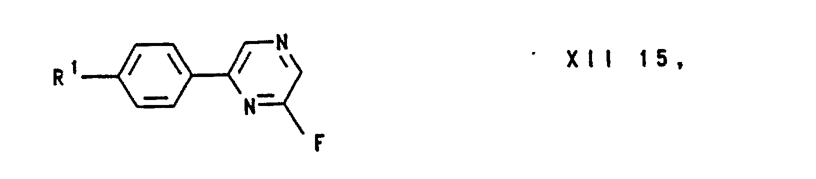 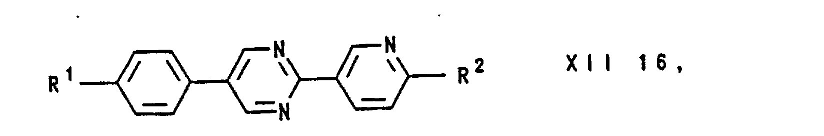 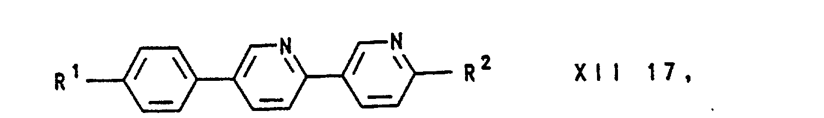 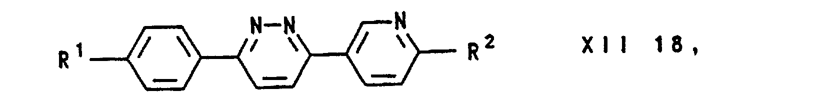 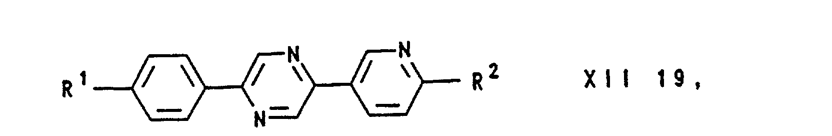 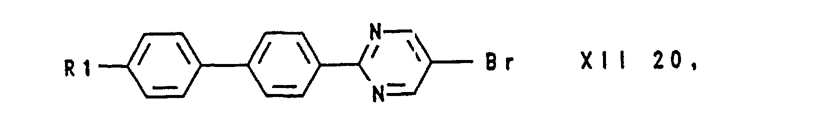 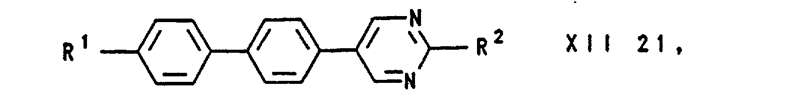 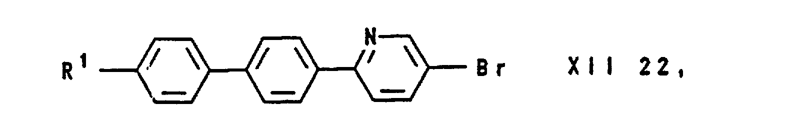 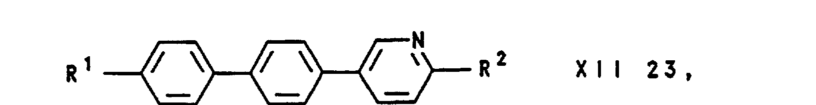 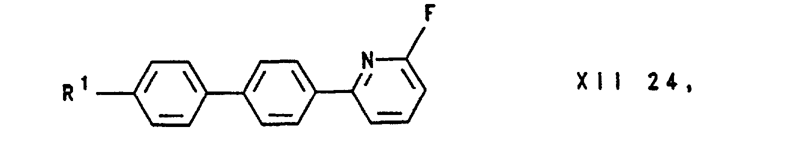 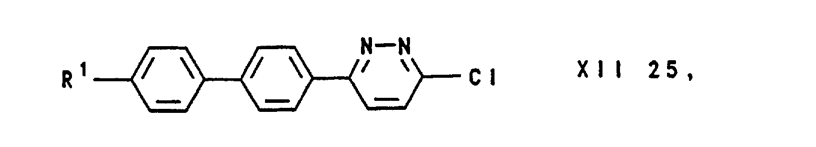 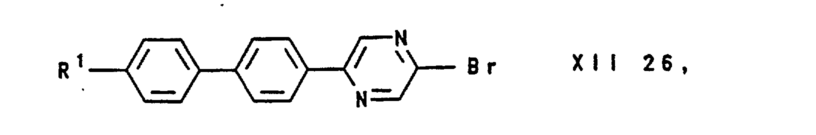 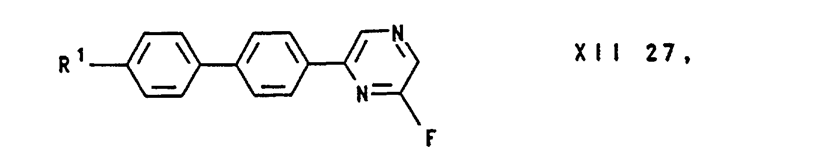 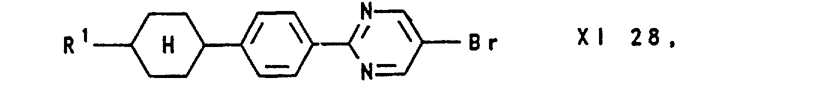 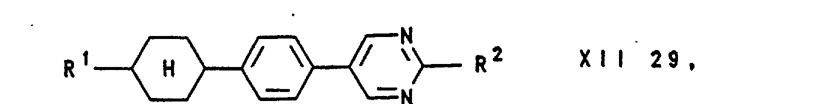 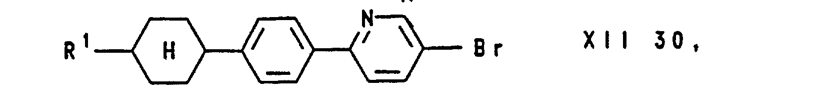 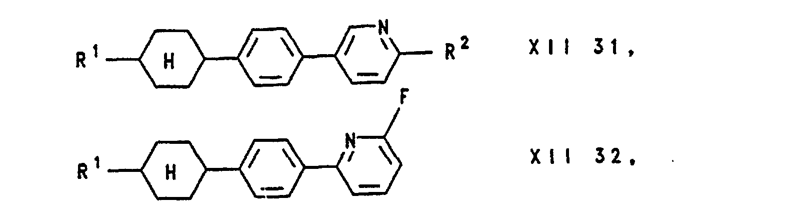 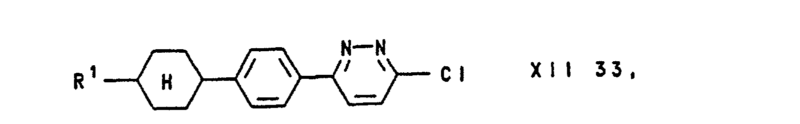 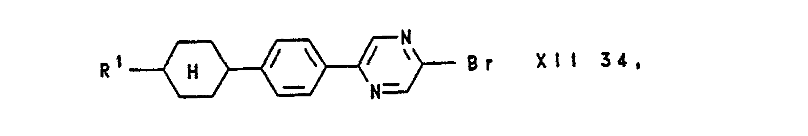 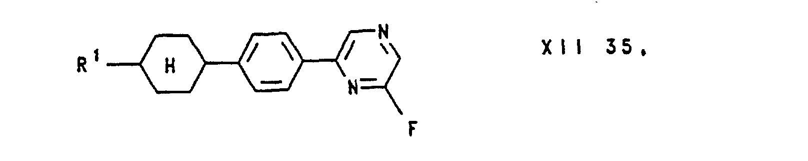 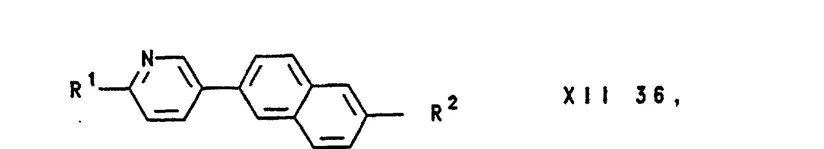 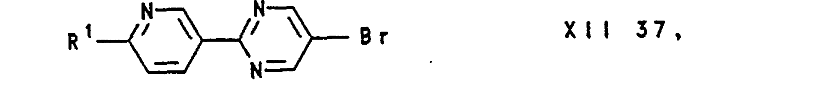 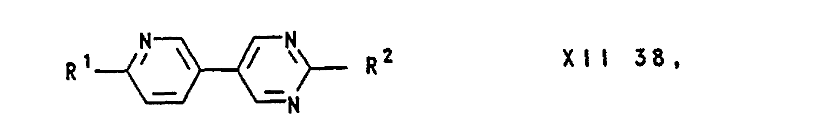 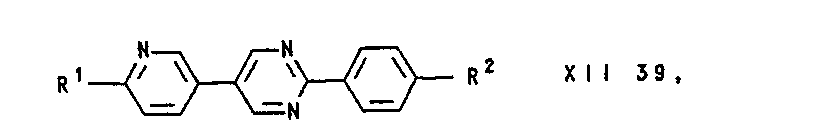 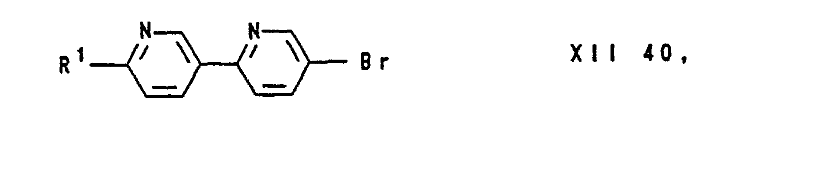 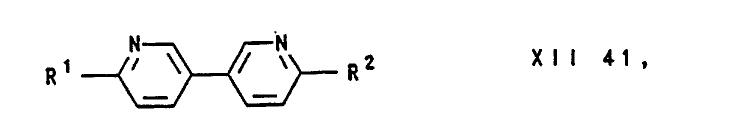 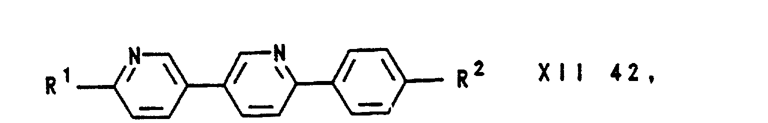 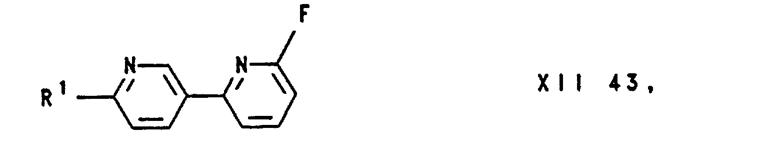 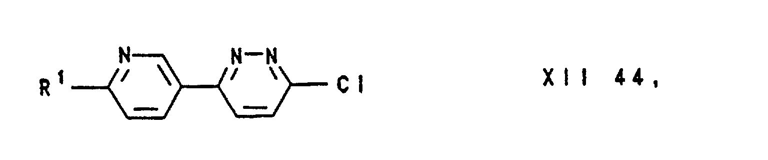 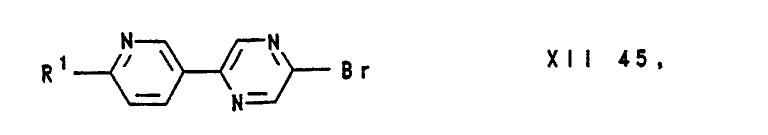 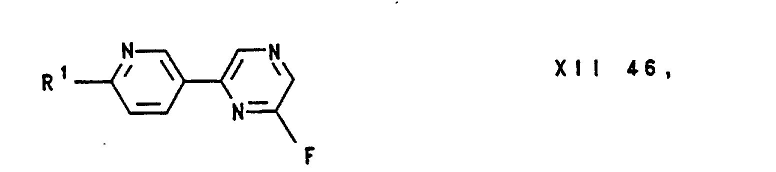 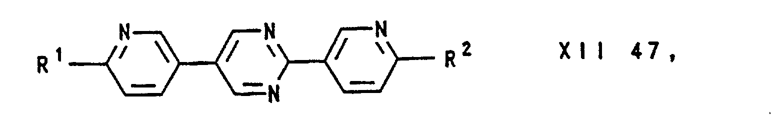 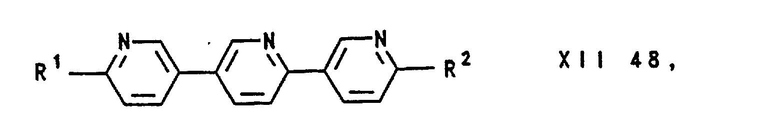 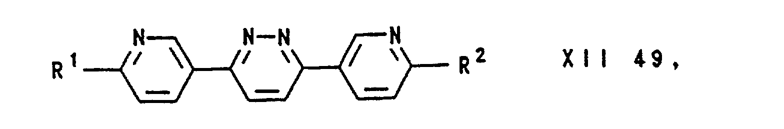 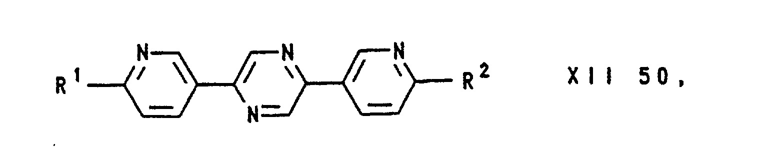 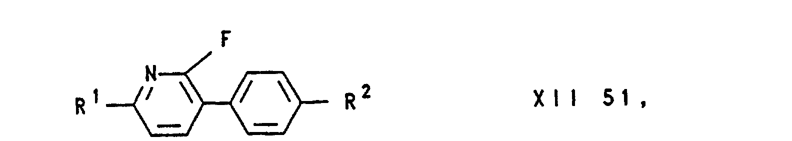 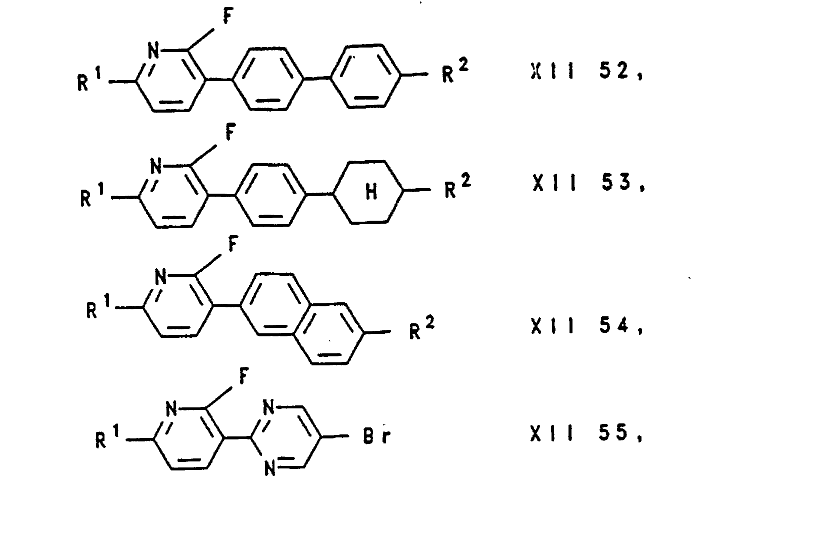 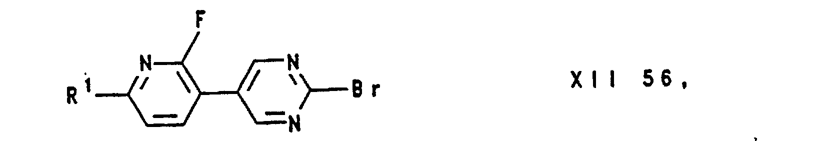 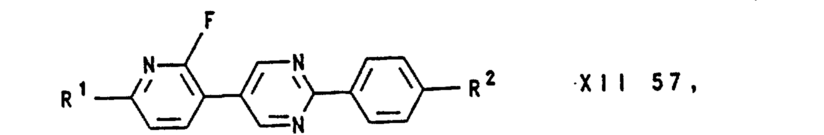 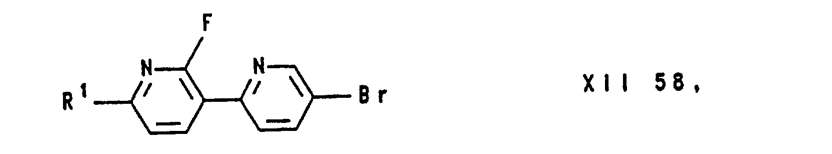 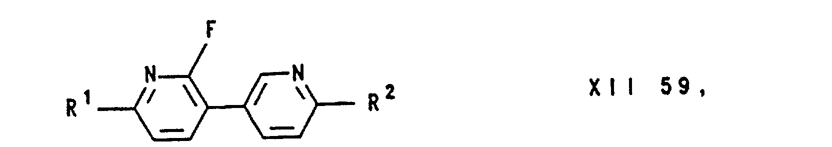 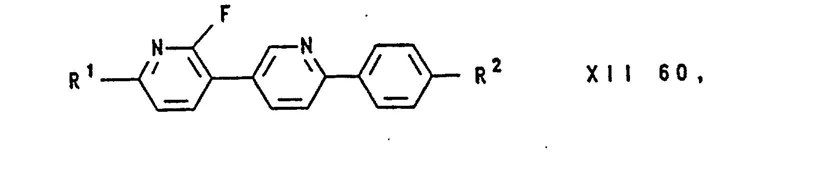 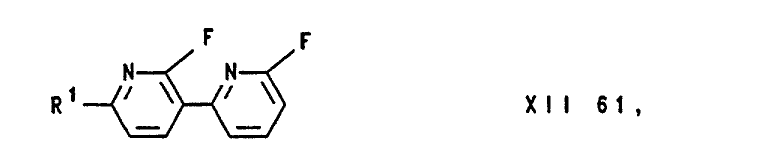 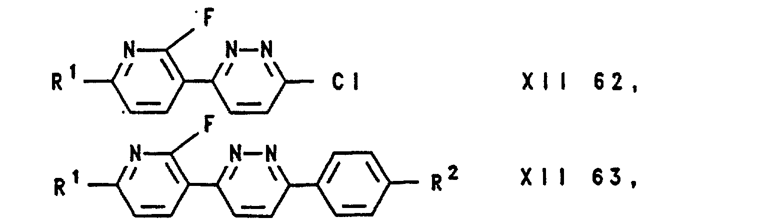 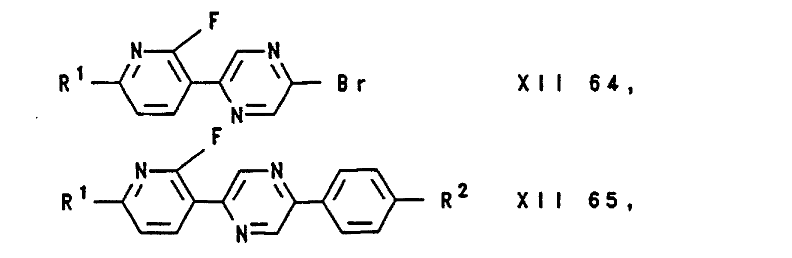 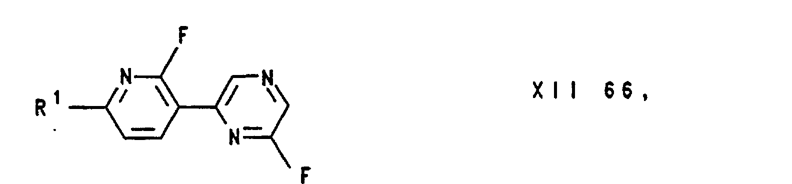 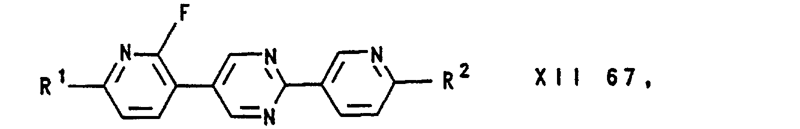 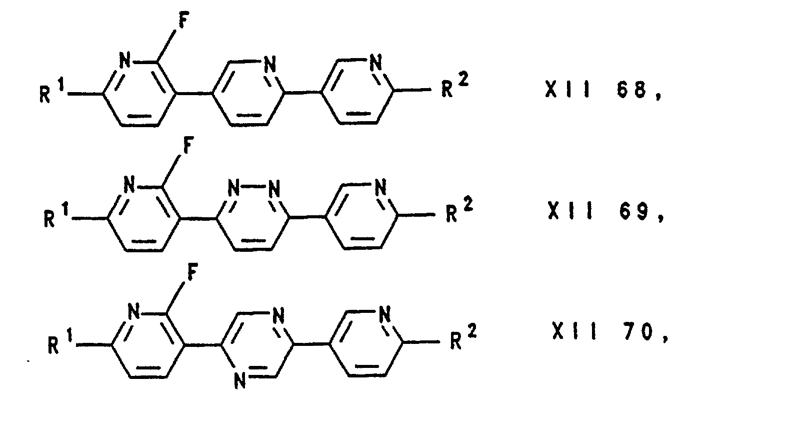 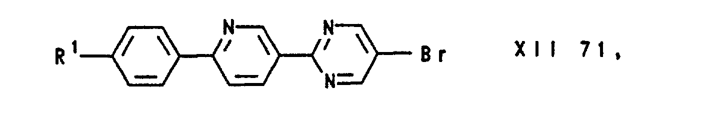 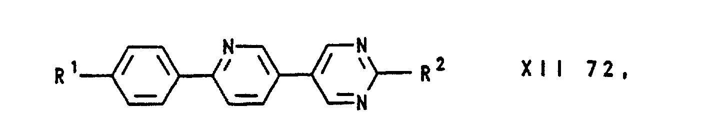 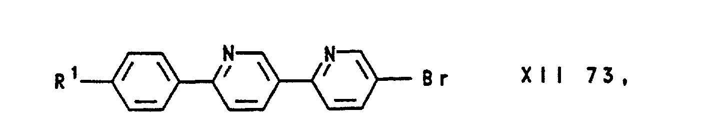 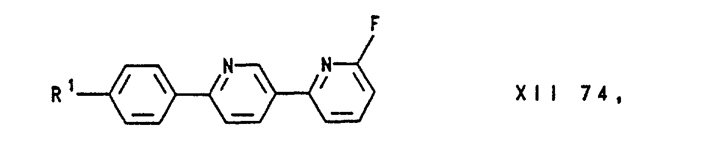 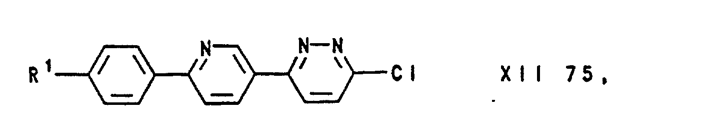 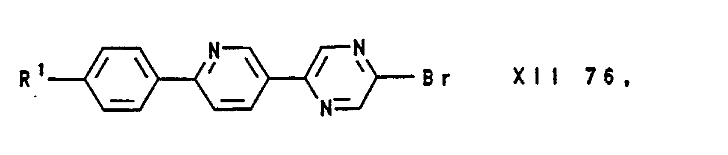 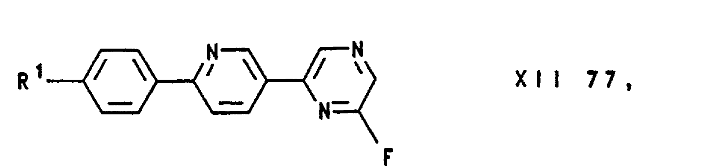 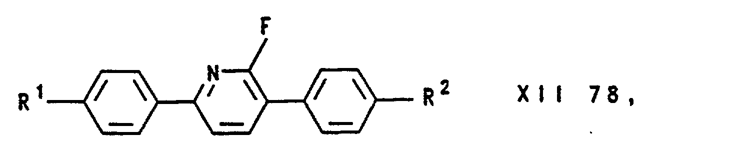 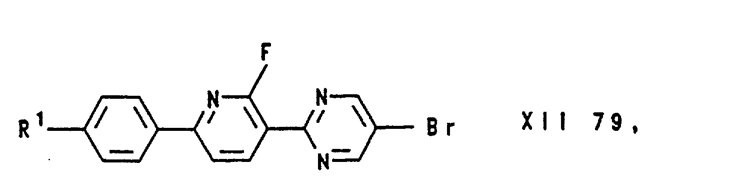 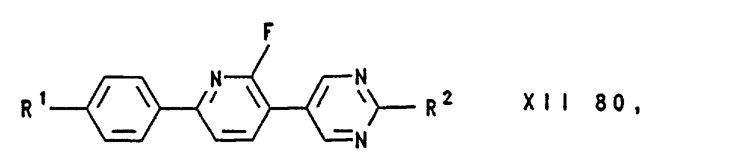 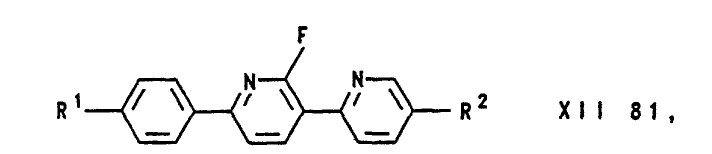 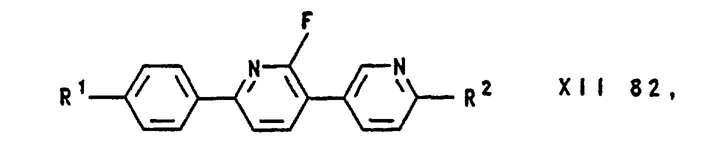 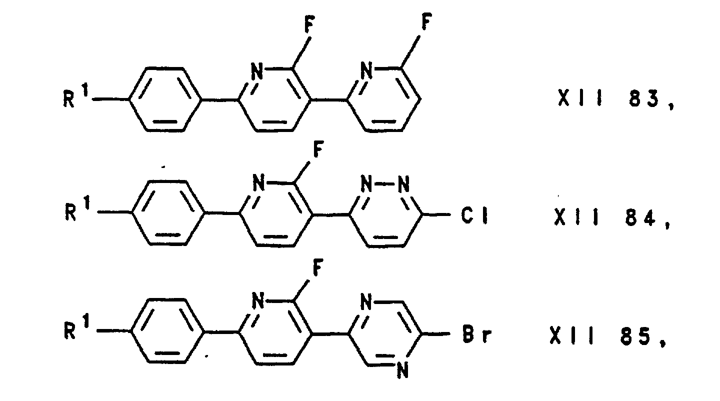 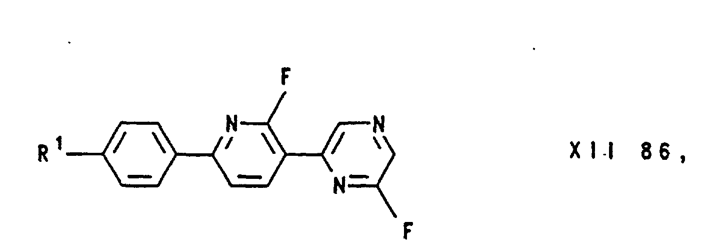 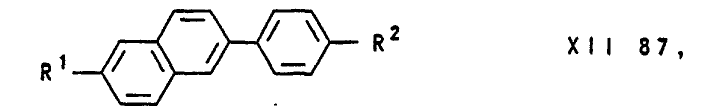 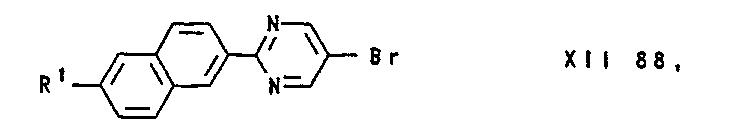 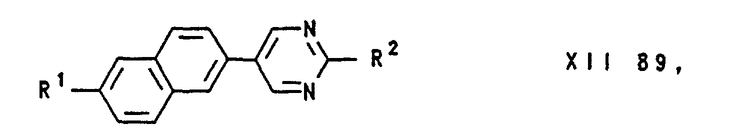 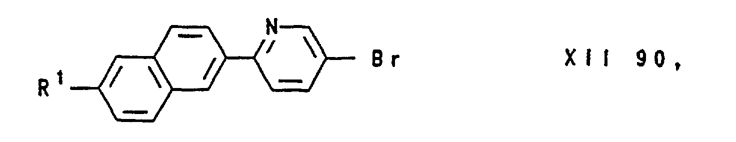 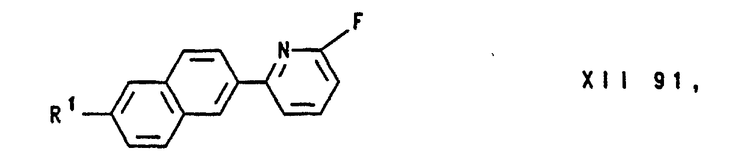 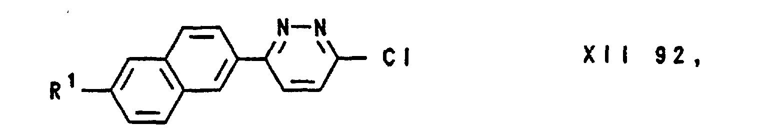 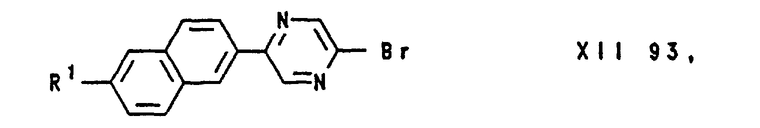 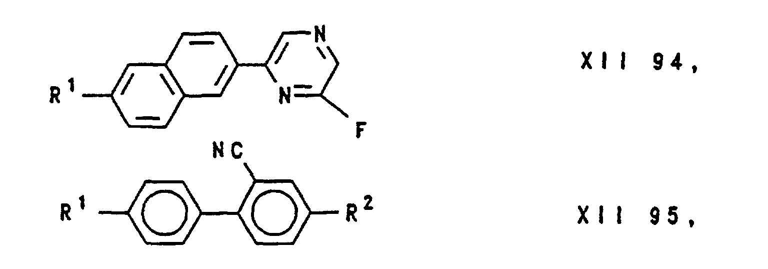 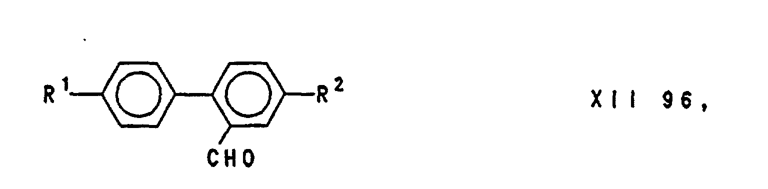 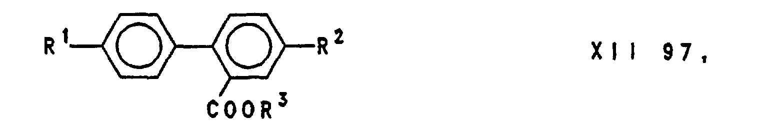 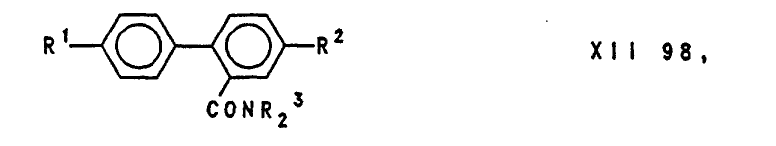 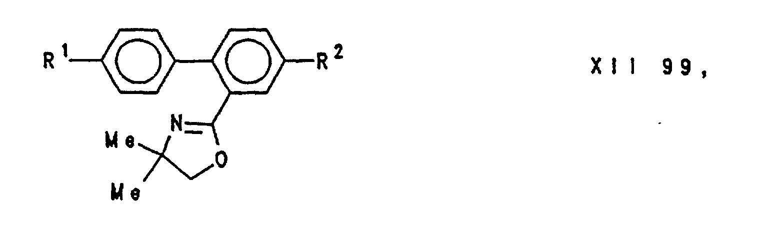 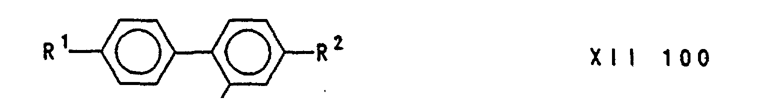 wobei R¹, R² und R³ Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und Pentadecyl, sowie Methoxy, Ethoxy, Propoxy, Butoxy, Pentoxy, Hexoxy, Heptoxy, Octoxy, Nonoxy, Decoxy, Undecoxy, Dodecoxy, Tridecoxy, Tetradecoxy und Pentadecoxy bedeuten.

Die Verbindungen der Formel XII eignen sich zum Einsatz als flüssigkristalline Materialien, oder können als Zwischenprodukte für die Herstellung weiterer flüssigkristalliner Verbindungen verwendet werden. Desweiteren werden Verbindungen der Formel I als Vorprodukte für Pharmazeutika, Kosmetika, Fungizide, Herbizide, Insektizide, Farbstoffe, Detergenzien und Polymere, einschließlich als Zusatzstoffe derselben, eingesetzt.

Erfindungsgemäß hergestellte Verbindungen, wie sie beispielsweise durch die Formeln XII 95 bis XII 100 wiedergegeben werden, sind insbesondere wertvolle Vorstufen für die Angiotensin II Inhibitoren (siehe z.B. WO-A 89/12 1201).

Die vorliegende Erfindung soll durch die nachfolgend beschriebenen Beispiele näher erläutert werden, ohne sie dadurch zu begrenzen. Die verwendeten Abkürzungen haben dabei folgende Bedeutung:
- Fp.: = Schmelzpunkt
- X: = kristallin
- S: = smektisch
- S_{C}: = smektisch C
- S_{A}: = smektisch A
- N: = nematisch
- I: = isotrop

### Beispiel 1

2-Brombenzonitril (3,1 g) und 2,75 g 4-Methylphenylboronsäure werden in 20 ml Toluol und 10 ml Ethanol gelöst. Man versetzt die Lösung mit 0,18 g (1 Mol-%) Palladium auf Aktivkohle (10 Gew.-%) und 4,47 g Natriumcarbonat in 10 ml Wasser. Anschließend gibt man 0,36 mmol 3,3',3"-Triphenylphosphinotrisulfonylnatrium (TPPTS) zu. Die Mischung wird 12 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert (2,9 g 4-Methyl-2'-cyanobiphenyl). Schmelzpunkt 49°C

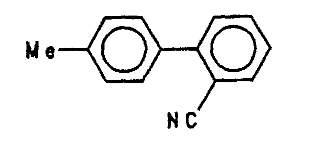

### Beispiel 2

2-Brombenzonitril (3,1 g) und 2,75 g 4-Methylphenylboronsäure werden in 20 ml Toluol und 10 ml Ethanol gelöst. Man versetzt die Lösung mit 0,018 g (0,1 Mol-%) Palladium auf Aktivkohle (10 Gew.-%) und 4,47 g Natriumcarbonat in 10 ml Wasser. Anschließend gibt man 0,036 mmol 3,3',3"-Triphenylphosphinotrisulfonylnatrium (TPPTS) zu. Die Mischung wird 18 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert (2,7 g 4-Methyl-2'-cyanobiphenyl).

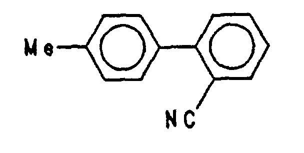

### Beispiel 3

2-Brombenzonitril (3,1 g) und 2,75 g 4-Methylphenylboronsäure werden in 20 ml Toluol und 10 ml Ethanol gelöst. Man versetzt die Lösung mit 0,18 g (1 Mol-%) Palladium auf Aktivkohle (10 Gew.-%) und 4,47 g Natriumcarbonat in 10 ml Wasser. Anschließend gibt man 0,18 mmol BINAS zu. Die Mischung wird 12 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert (2,55 g 4-Methyl-2'-cyanobiphenyl).

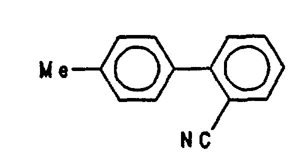

### Beispiel 4

2-Brombenzonitril (3,1 g) und 2,75 g 4-Methylphenylboronsäure werden in 20 ml Toluol und 10 ml Ethanol gelöst. Man versetzt die Lösung mit 0,018 g (0,1 Mol-%) Palladium auf Aktivkohle (10 Gew.-%) und 4,47 g Natriumcarbonat in 10 ml Wasser. Anschließend gibt man 0,018 mmol BINAS zu. Die Mischung wird 12 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert (2,45 g 4-Methyl-2'-cyanobiphenyl).

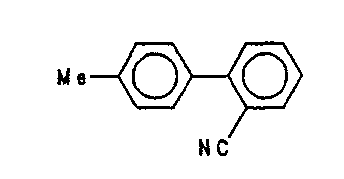

### Beispiel 5

2-Brombenzonitril (25,77 g) und 22,86 g 4-Methylphenylboronsäure werden in 166 ml Toluol und 83 ml Ethanol gelöst. Man versetzt die Lösung mit 0,15 g (0,1 Mol-%) Palladium auf Aktivkohle (10 Gew.-%) und 37,16 g Natriurncarbonat in 83 ml Wasser. Anschließend gibt man 2,83 mmol TPPTS zu. Die Mischung wird 18 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 500 ml n-Heptan kristallisiert (24,5 g 4-Methyl-2'-cyanobiphenyl).

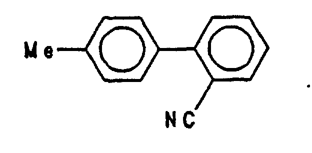

### Beispiel 6

10,43 g (0,044 Mol) 2,5-Dibrompyrimidin, 10 (0,044 Mol) 4-(Phenylmethoxy)-benzolboronsäure, 0,475 g (0,00044 Mol) Palladium auf Aktivkohle (10 Gew.-%), (0,001752 Mol) TPPTS und 9,3 g (0,0876 Mol) Natriumcarbonat werden in 100 ml Toluol, 50 ml Ethanol und 30 ml Wasser für 48 h auf 80°C erhitzt. Anschließend wird bei 80°C der Palladiumkatalysator durch Filtration vom Reaktionsgemisch abgetrennt. Die wäßrige Unterphase des Reaktionsgemisches wird bei 80°C abgetrennt bevor die organische Phase am Rotationsverdampfer von den Lösungsmitteln befreit und im Hochvakuum getrocknet wird. Das so erhaltene Rohprodukt wird aus Acetonitril (300 ml) kristallisiert, wonach 14,5 g (93 % Ausbeute, bezogen auf 2,5-Dibrompyrimidin) 5-Brom-2-[4-(phenylmethoxy)phenyl]pyrimidin (Gehalt nach HPLC 98 %) erhalten werden.

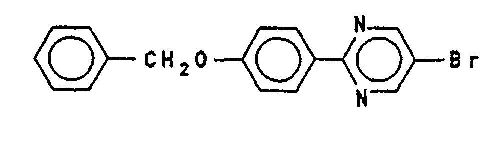

### Beispiel 7

Analog Beispiel 6 aus 27,7 g (100 mmol) 5-[1,3,2]Dioxaborolan-2-yl-2-octyloxy-pyridin und 28,9 g (100 mmol) 5-Brom-2-octyloxypyrimidin, 0,53 g (0,5 mmol) Palladium auf Aktivkohle, 2 mmol BINAS und 21,2 g (200 mmol) Natriumcarbonat. Nach Chromatographie an Kieselgel erhält man 39,7 g (96 %) Produkt.

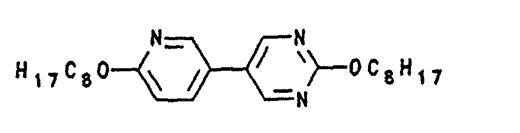

| | | | | | | | |
|---|---|---|---|---|---|---|---|
| Phasenfolge | X | 64 | S_{C} | 67 | S_{A}91 | 91 | l |

### Beispiel 8

Analog Beispiel 6 aus 24,3 g (100 mmol) 2-Brom-5-hexylpyrimidin und 21,9 g (100 mmol) 4-Hexyloxybenzolboronsäure, 0,53 g (0,5 mmol) Palladium auf Aktivkohle, 2 mmol BINAS und 21,2 g (200 mmol) Natriumcarbonat. Nach Chromatographie an Kieselgel erhält man 32,3 g (95 %) Produkt.

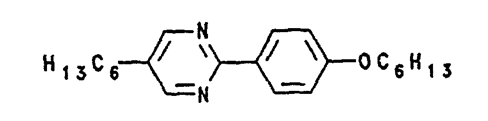

| | | | | | | | |
|---|---|---|---|---|---|---|---|
| Phasenfolge | X₁ | 15 | X₂ | 32 | N | 61 | l |

### Beispiel 9

2-Brombenzonitril (3,1 g) und 3,9 g p-(Benzyloxy)phenylboronsäure werden in 20 ml Toluol und 10 ml Ethanol gelöst. Man versetzt die Lösung mit 0,18 g (1 Mol-%) Palladium auf Aktivkohle (10 Gew.-%) und 4,47 g Natriumcarbonat in 10 ml Wasser. Anschließend gibt man 0,36 mmol TPPTS zu. Die Mischung wird 12 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert (4,36 g 4-(Benzyloxy)-2'-cyano-biphenyl).

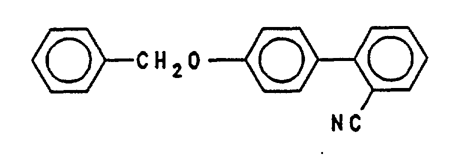

### Beispiel 10

2-Brombenzonitril (3,1 g) und 3,9 g p-(Benzyloxy)phenylboronsäure werden in 20 ml Toluol und 10 ml Ethanol gelöst. Man versetzt die Lösung mit 0,18 g (1 Mol-%) Palladium auf Aktivkohle (10 Gew.-%) und 4,47 g Natriumcarbonat in 10 ml Wasser. Anschließend gibt man 0,18 mmol BINAS zu. Die Mischung wird 12 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert (4,3 g 4-(Benzyloxy)-2'-cyano-biphenyl).

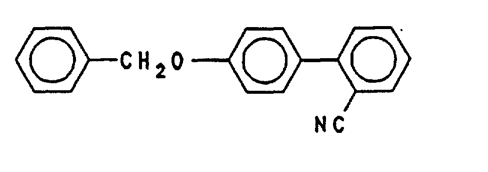

## Patentansprüche

1. Verfahren zur Herstellung mehrkerniger aromatischer Verbindungen durch Kreuzkupplung von aromatischen Borverbindungen mit aromatischen Halogenverbindungen oder - Perfluoralkylsulfonaten in Gegenwart von metallischem Palladium als Katalysator und soviel Wasser, daß die Reaktionsmischung eine wäßrige Phase ausbildet, **dadurch gekennzeichnet, daß** man der Reaktion mindestens einen wasserlöslichen Komplexliganden zusetzt.

2. Verfahren nach Anspruch 1, **dadurch gekennzeichnet, daß** eine mehrkernige aromatische Verbindung der Formel XII hergestellt wird,
R¹(-A¹)ₖ(-M¹)ₗ-A²-A³(-M²)ₘ(-A⁴)ₙ-R² (XII)
wobei R¹ und R² unabhänigig voneinander Benzyloxy, H, F, Cl, Br, -NC, -CN, -CF₃, -OCF₃ oder ein geradkettiger oder verzweigter (mit oder ohne asymmetrisches C-Atom) Alkylrest mit 1 bis 18 C-Atomen sein können, wobei auch eine oder zwei nicht benachbarte -CH₂-Gruppen durch -O-, -S-, -CO-, -CO-O-, -O-CO-, -CO-S-, -S-CO-, -O-CO-O-, -CH=CH-, -C≡C-, -SO₂-, CON(H,C₁-C₈-Alkyl), 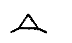 oder -Si(CH₃)₂- ersetzt sein können, und wobei auch ein oder mehrere H-Atome des Alkylrestes durch F, Cl, Br oder CN substituiert sein können,
A¹ und A⁴ jeweils unabhängig voneinander 1,4-Phenylen, Pyrazin-2,5-diyl, Pyridazin-3,6-diyl, Pyridin-2,5-diyl, Pyrimidin-2,5-diyl, Naphthalin-2,6-diyl, trans-1,4-Cyclohexylen, worin eine oder zwei nicht benachbarte CH₂-Gruppen durch -O- oder -S- ersetzt sein können und wobei ein oder mehrere H-Atome gleich oder verschieden durch Substituenten L ersetzt sein können, wobei L die unter R¹ genannten Bedeutungen hat oder 4,4-Dimethylisoxazolin ist, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Bicyclo[2.2.2]octan-1,4-diyl, 1,3-Dioxaborinan-2,5-diyl oder trans-Dekalin-2,6-diyl,
A² und A³ jeweils unabhänig voneinander 1,4-Phenylen, Pyrazin-2,5-diyl, Pyridazin-3,6-diyl, Pyridin-2,5-diyl, Pyrimidin-2,5-diyl, Naphthalin-2,6-diyl, wobei ein oder mehrere H-Atome gleich oder verschieden durch Substituenten L ersetzt sein können, wobei L die unter R¹ genannten Bedeutungen hat, 4,4-Dimethylisoxazolin oder im Falle von A³ auch CHO ist, 1,3,4-Thiadiazol-2,5-diyl, 1,3-Thiazol-2,4-diyl, 1,3-Thiazol-2,5-diyl, Thiophen-2,4-diyl oder Thiophen-2,5-diyl sein können,
M¹ und M² jeweils unabhänig voneinander
-O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-, -CH₂CH₂CH₂-O-, -OCH₂CH₂CH₂-, -CH₂CH₂CO-O-, -O-COCH₂CH₂-sein können,
k, l, m, n jeweils unabhängig voneinander Null oder Eins bedeuten, indem man eine aromatische Borverbindung der Formel (VII)
R¹(-A¹)ₖ(-M¹)ₗ-A²-BQ₁Q₂ (VII),
R¹, A¹, A², M¹, k und l wobei die oben angegebene Bedeutung haben,
Q_{1,} Q₂ gleich oder verschieden -OH, C₁-C₄-Alkoxy, C₁-C₄-Alkyl, Phenyl, das gegebenenfalls durch C₁-C₄-Alkyl, C₁-C₄-Alkoxy oder Halogen substituiert sein kann, oder Halogen bedeuten oder Q₁ und Q₂ zusammen bilden eine C₁-C₄-Alkylendioxy-Gruppe, eine Methylengruppe, die gegebenenfalls durch eine oder zwei C₁-C₄-Alkylgruppen substituiert sein kann, oder Q₁ und Q₂ und das Boratom zusammen sind Teil einer Boroxinrings der Formel (IX): 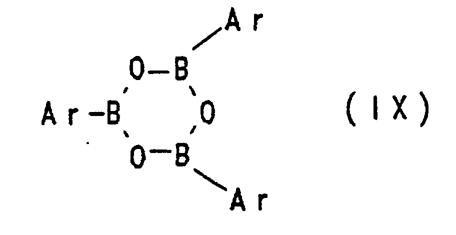
mit einer Verbindung der Formel (XI) umsetzt,
X-A³(-M²)ₘ(-A⁴)ₙ-R² (XI),
wobei R², R³, A³, A⁴, M², m und n die oben angegebene Bedeutung haben, und X Chlor, Brom, Jod oder OSO₂-CₚF₂ₚ₊₁, worin p einen ganzzahligen Wert von 1 bis 10 darstellt, bedeutet.

3. Verfahren nach Anspruch 1 oder 2, **dadurch gekennzeichnet, daß** als wasserlöslicher Komplexligand mindestens eine Verbindung aus der Gruppe Phosphane, Phosphite, Phophonigsäureester, Phosphinigsäureester, Phosphole, Bipyridine, Phenanthroline, Porphyrine und Alizarine verwendet wird.

4. Verfahren nach Anspruch 3, **dadurch gekennzeichnet, daß** der wasserlösliche Ligand in einem Anteil von 0,1-20 Mol-%, bezogen auf die aromatische Halogenverbindung oder das aromatische Perfluoralkylsulfonat, zugesetzt wird.

5. Verfahren nach einem oder mehreren der vorhergehenden Ansprüche, **dadurch gekennzeichnet, daß** als Base mindestens eine Verbindung aus der Gruppe Alkali und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkloholate und primäre, sekundäre und tertiäre Amine verwendet wird.

6. Verfahren nach Anspruch 5, **dadurch gekennzeichnet, daß** die Base in einem Anteil von 100-500 Mol.-%, bezogen auf die aromatische Boronsäure, verwendet wird.

7. Verfahren nach einem oder mehreren der vorhergehenden Ansprüche, **dadurch gekennzeichnet, daß** es bei Temperaturen zwischen 50 und 150°C durchgeführt wird.

8. Verfahren nach einem oder mehreren der vorhergehenden Ansprüche, **dadurch gekennzeichnet, daß** die Kreuzkupplungsreaktion in einem Lösungsmittelgemisch durchgeführt wird, das neben Wasser mindestens eine Verbindung aus der Gruppe Ether, Kohlenwasserstoffe, Alkohole, Ketone, Amide und Nitrile enthält.

9. Verfahren nach einem oder mehreren der vorhergehenden Ansprüche, **dadurch gekennzeichnet, daß** als Katalysator Palladium in pulverisierter Form, Palladium auf Aktivkohle, Palladium auf Aluminiumoxid, Palladium auf Bariumsulfat oder Palladium auf Calciumcarbonat, jeweils mit einem Palladiumgehalt von 0,5 bis 10 Gew.-%, verwendet wird.

## Claims

1. Process for the production of polycyclic aromatic compounds by cross-coupling aromatic boron compounds with aromatic halogen compounds or perfluoroalkyl sulfonates in the presence of metallic palladium as catalyst and a quantity of water sufficient for the reaction mixture to form an aqueous phase, **characterised in that** at least one water-soluble complex ligand is added to the reaction.

2. Process according to claim 1, **characterised in that** a polycyclic aromatic compound of the formula XII is produced
R¹(-A¹)ₖ(-M¹)ₗ-A²-A³(-M²)ₘ(-A⁴)ₙ-R² (XII),
wherein R¹ and R² may mutually independently be benzyloxy, H, F, Cl, Br, -NC, -CN, -CF₃, -OCF₃ or a linear or branched alkyl residue (with or without an asymmetrical C atom) having 1 to 18 C atoms, wherein one or two non-adjacent -CH₂ groups may be replaced by -O-, -S-, -CO-, -CO-O-, -O-CO-, -CO-S-, -S-CO-, -O-CO-O-, -CH=CH-, -C≡C-, -SO₂-, CON (H, C₁-C₈ alkyl), 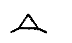 or -Si(CH₃)₂, and wherein one or more H atoms of the alkyl residue may also be substituted by F, Cl, Br or CN,
A¹ and A⁴ may each mutually independently be 1,4-phenylene, pyrazine-2,5-diyl, pyridazine-3,6-diyl, pyridine-2,5-diyl, pyrimidine-2,5-diyl, naphthalene-2,6-diyl, trans-1,4-cyclohexylene, in which one or two non-adjacent CH₂ groups may be replaced by -O- or -S- and wherein one or more H atoms may be replaced identically or differently by substituents L, wherein L has the meanings stated for R¹ or is 4,4-dimethylisoxazoline, (1,3,4)-thiadiazole-2,5-diyl, 1,3-thiazole-2,4-diyl, 1,3-thiazole-2,5-diyl, thiophene-2,4-diyl, thiophene-2,5-diyl, piperazine-1,4-diyl, piperazine-2,5-diyl, piperidine-1,4-diyl, bicyclo[2.2.2]octane-1,4-diyl, 1,3-dioxaborinane-2,5-diyl or trans-decalin-2,6-diyl,
A² and A³ may each mutually independently be 1,4-phenylene, pyrazine-2,5-diyl, pyridazine-3,6-diyl, pyridine-2,5-diyl, pyrimidine-2,5-diyl, naphthalene-2,6-diyl, wherein one or more H atoms may be replaced identically or differently by substituents L, wherein L has the meanings stated for R¹, is 4,4-dimethylisoxazoline or, in the case of A³, also CHO, 1,3,4-thiadiazole-2,5-diyl, 1,3-thiazole-2,4-diyl, 1,3-thiazole-2,5-diyl, thiophene-2,4-diyl or thiophene-2,5-diyl,
M¹ and M² may each mutually independently be -O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-, -CH₂CH₂CH₂-O-, -OCH₂CH₂CH₂-, -CH₂CH₂CO-O-, -O-COCH₂CH₂-,
k, l, m, n each mutually independently mean zero or one,
by reacting an aromatic boron compound of the formula (VII)
R¹(-A¹)ₖ(-M¹)ₗ-A²-BQ₁Q₂ (VII),
wherein R¹, A¹, A², M¹, k and l have the above-stated meaning,
Q₁, Q₂ mean identically or differently -OH, C₁-C₄ alkoxy, C₁-C₄ alkyl, phenyl, which may optionally be substituted by C₁-C₄ alkyl, C₁-C₄ alkoxy or halogen, or halogen or Q₁ and Q₂ together form a C₁-C₄ alkylenedioxy group, a methylene group, which may optionally be substituted by one or two C₁-C₄ alkyl groups, or Q₁ and Q₂ and the boron atom are together part of a boroxine ring of the formula (IX): 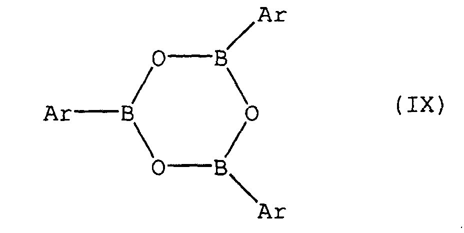 with a compound of the formula (XI)
X-A³(-M²)ₘ(-A⁴)ₙ-R² (XI),
wherein R², R³, A³, A⁴, M², m and n have the above-stated meaning, and X means chlorine, bromine, iodine or OSO₂-CₚF₂ₚ₊₁, in which p is an integer from 1 to 10.

3. Process according to claim 1 or 2, **characterised in that** the water-soluble complex ligand used comprises at least one compound from the group of phosphanes, phosphites, phosphonous acid esters, phosphinous acids esters, phospholes, bipyridines, phenanthrolines, porphyrins and alizarins.

4. Process according to claim 3, **characterised in that** the water-soluble ligand is added in a proportion of 0.1-20 mol%, relative to the aromatic halogen compound or the aromatic perfluoroalkyl sulfonate.

5. Process according to one or more of the preceding claims, **characterised in that** the base used comprises at least one compound from the group of alkali metal and alkaline earth metal hydroxides, alkali metal and alkaline earth metal carbonates, alkali metal hydrogen carbonates, alkali metal and alkaline earth metal acetates, alkali metal and alkaline earth metal alkoxides and primary, secondary and tertiary amines.

6. Process according to claim 5, **characterised in that** the based is used in a proportion of 100-500 mol%, relative to the aromatic boronic acid.

7. Process according to one or more of the preceding claims, **characterised in that** it is performed at temperatures of between 50 and 150°C.

8. Process according to one or more of the preceding claims, **characterised in that** the cross-coupling reaction is performed in a solvent mixture which contains, apart from water, at least one compound from the group of ethers, hydrocarbons, alcohols, ketones, amides and nitriles.

9. Process according to one or more of the preceding claims, **characterised in that** the catalyst used comprises palladium in pulverised form, palladium on activated carbon, palladium on aluminium oxide, palladium on barium sulfate or palladium on calcium carbonate, in each case with a palladium content of 0.5 to 10 wt.%.

## Revendications

1. Procédé pour la préparation de composés aromatiques à plusieurs noyaux par couplage croisé de composés aromatiques de bore avec des composés aromatiques halogénés ou des perfuoroalkylsulfonates, en présence de palladium métal en tant que catalyseur et d'une quantité d'eau telle que le mélange réactionnel forme une phase aqueuse, **caractérisé en ce qu'**on ajoute à la réaction au moins un ligand complexe hydrosoluble.

2. Procédé selon la revendication 1, **caractérisé en ce qu'**on prépare un composé aromatique à plusieurs noyaux de formule XII,
R¹(-A¹)ₖ(-M¹)ₗ-A²-A³(-M²)ₘ(-A⁴)ₙ-R² (XII)
où R¹ et R² peuvent représenter, indépendamment l'un de l'autre, des groupes benzyloxy, H, F, Cl, Br, -NC, -CN, -CF₃, -OCF₃ ou un reste alkyle à chaîne droite ou ramifiée (avec ou sans atome de carbone asymétrique) comportant 1 à 18 atomes de carbone, un ou deux groupes -CH₂- non adjacents pouvant être remplacés par des groupes -O-, -S-, -CO-, -CO-O-, -O-CO-, -CO-S-, -S-CO-, -O-CO-O-, -CH=CH-, -C≡C-, -SO₂-, CONH(H,alkyle en C₁-C₈), 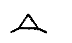 ou -Si(-CF₃)₂-, et un ou plusieurs atomes de H du reste alkyle pouvant être substitués par F, Cl, Br ou CN,
A¹ et des groupes 1,4-phénylène, pyrazin-2,5-diyle, pyridazine-3,6-diyle, pyridine-2,5-diyle, pyrimidine-2,5-diyle, naphtalène-2,6-diyle, trans-1,4-cyclohexylène, où un ou deux groupes CH₂ non adjacents peuvent être remplacés par groupes -O- ou -S- et un ou plusieurs atomes de H pouvant être remplacés par des substituants L identiques ou différents, L possédant la signification donnée à R¹ ou représente des groupes 4,4-diméthylisoxazoline, (1,3,4)-thiadiazol-2,5-diyle, 1,3-thiazol-2,4-diyle, 1,3-thiazol-2,5-diyle, thiophène-2,4-diyle, thiophène-2,5-diyle, pipérazine-1,4-diyle, pipérazine-2,5-diyle, pipéridine-1,4-diyle, bicyclo[2.2.2]octane-1,4-diyle, 1,3-dioxaborinane-2,5-diyle ou trans-décaline-2,6-diyle,
A² et A³ représentent chacun, indépendamment l'un de l'autre, des groupes 1,4-phénylène, pyrazin-2,5-diyle, pyridazine-3,6-diyle, pyridine-2,5-diyle, pyrimidine-2,5-diyle, naphtalène-2,6-diyle, un ou plusieurs atomes de H pouvant être remplacés par des substituants L identiques ou différents, L possédant les significations données à R¹, 4,4-diméthyisoxazoline ou dans le cas de A³ représentant aussi CHO, 1,3,4-thiadiazol-2,5-diyle, 1,3-thiazol-2,4-diyle, 1,3-thiazol-2,5-diyle, thiophène-2,4-diyle ou thiophène-2,5-diyle,
M¹ et M² représentent chacun, indépendamment l'un de l'autre, -O-, -S-, -CO-, -CO-O-, -O-CO-, -CO-S-, -S-CO-, -O-CO-O-, -CH₂-O-, -OCH₂-, -CH₂-CH₂-, -CH=CH-, -C≡C-, -CH(CN)-CH₂-, -CH₂-CH(CN)-, -CH=N-, -N=CH-, -CH₂CH₂CH₂-O-, -O-CH₂CH₂CH₂-, -CH₂CH₂-CO-O-, -O-COCH₂CH₂-,
k, l, m, n valent chacun, indépendamment l'un de l'autre, zéro ou un,
en faisant réagir un composé aromatique de bore de formule (VII)
R¹(-A¹)ₖ(-M)ₗ-A²-BQ₁Q₂)ₙ-R² (VII)
où R¹, A¹, A², M¹, k et l possèdent la signification donnée ci-dessus,
Q₁, Q₂ sont identiques ou différentes et représentent des groupes -OH, alkoxy en C₁-C₄, alkyle en C₁-C₄, phényle, qui peut être éventuellement substitué par un substituant alkyle en C₁-C₄, alkoxy en C₁-C₄ ou halogène, ou représentent un atome d'halogène, ou Q₁ et Q₂ ensemble forment un groupe (alkylène en C₁-C₄)dioxy, un groupe méthylène, qui peut être éventuellement substitué par un ou deux groupes alkyle en C₁-C₄, ou Q₁ et Q₂ et l'atome de bore font partie d'un cycle boroxine.de formule (IX) 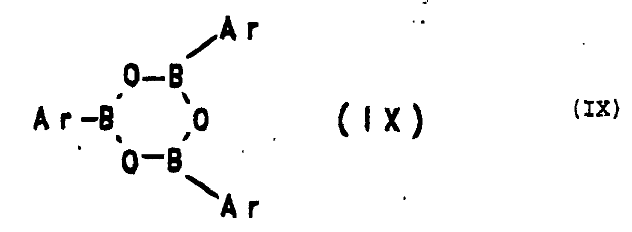
avec un composé de formule (XI),
X-A³(-M²)ₘ(-A⁴)ₙ-R² (XI),
où R², R³, A³, A⁴, M², m et n possédent les significations données ci-dessus, et X représente un atome de chlore, de brome, d'iode ou OSO₂-CₚF₂ₚ₊₁, où p est un entier de 1 à 10.

3. Procédé selon la revendication 1 ou 2, **caractérisé en ce qu'**on utilise en tant que ligand complexe hydrosoluble au moins un composé pris dans le groupe comprotant des phosphanes, phosphites, esters d'acide phosphoneux, esters d'acide phosphineux, phosphols, bipyridines, phénanthrolines, porphyrines et alizarines.

4. Procédé selon la revendication 3, **caractérisé en ce qu'**on ajoute le ligand hydrosoluble en un taux de 0,1 à 20 % molaires, par rapport aux composé aromatique halogéné ou le perfluoralkylsulfonate.

5. Procédé selon une ou plusieurs des revendications précédentes, **caractérisé en ce qu'**on utilise comme base au moins un composé pris dans le groupe comportant des hydroxydes de métaux alcalins et alcalino-terreux, des carbonates de métaux alcalins et alcalino-terreux, des bicarbonates de métaux alcalins et alcalino-terreux, des acétates de métaux alcalins et alcalino-terreux, des alcoolates de métaux alcalins et alcalino-terreux et des amines primaires, secondaires et tertiaires.

6. Procédé selon la revendication 5, **caractérisé en ce qu'**on utilise la base en un taux de 100 à 500 % molaires, par rapport à l'acide boronique aromatique.

7. Procédé selon une ou plusieurs des revendications précédentes, **caractérisé en ce qu'**il est mis en oeuvre à une température comprise entre 50 et 150°C.

8. Procédé selon une ou plusieurs des revendications précédentes, **caractérisé en ce qu'**on met en oeuvre la réaction de couplage croisé dans un solvant, qui contient au côté d'eau, au moins un composé pris dans le groupe comportant des éthers, hydrocarbures, alcools, cétones, amides et nitriles.

9. Procédé selon une ou plusieurs des revendications précédentes, **caractérisé en ce qu'**on utilise comme catalyseur le palladium sous forme pulvérisée, le palladium sur le charbon actif, le palladium sur l'oxyde d'aluminium, le palladium sur le sulfate de baryum ou le palladium sur le carbonate de calcium, à chaque fois avec une teneur en palladium de 0,5 à 10 % en masse.
